(19) Europäisches Patentamt
European Patent Office
Office européen des brevets

(11) Veröffentlichungsnummer: **0 247 477 B1**

(12) # EUROPÄISCHE PATENTSCHRIFT

(45) Veröffentlichungstag der Patentschrift: **27.06.90**

(21) Anmeldenummer: **87107154.4**

(22) Anmeldetag: **18.05.87**

(51) Int. Cl.$^5$: **C 07 D 487/04,** A 01 N 43/90 // (C07D487/04, 239:00, 235:00),(C07D487/04, 239:00, 239:00),(C07D487/04, 243:00, 239:00)

(54) **1,2,3,6-Tetrahydro-5-nitro-pyrimidin-Derivate.**

(30) Priorität: **30.05.86 DE 3618126**
**08.11.86 DE 3638121**

(43) Veröffentlichungstag der Anmeldung:
**02.12.87 Patentblatt 87/49**

(45) Bekanntmachung des Hinweises auf die Patenterteilung:
**27.06.90 Patentblatt 90/26**

(84) Benannte Vertragsstaaten:
**AT BE CH DE ES FR GB GR IT LI NL**

(56) Entgegenhaltungen:
**EP-A-0 004 173**
**US-A-4 031 087**

(73) Patentinhaber: **BAYER AG**
**D-5090 Leverkusen 1 Bayerwerk (DE)**

(72) Erfinder: **Wolf, Hilmar, Dr.**
**Haus Gravener Strasse 105**
**D-4018 Langenfeld (DE)**
Erfinder: **Becker, Benedikt, Dr.**
**Metzkausener Strasse 14**
**D-4020 Mettmann (DE)**
Erfinder: **Homeyer, Bernhard, Dr.**
**Obere Strasse 28**
**D-5090 Leverkusen 3 (DE)**
Erfinder: **Stendel, Wilhelm, Dr.**
**In den Birken 55**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 247 477 B1

Courier Press, Leamington Spa, England.

**Beschreibung**

Die vorliegende Erfindung betrifft neue 1,2,3,6-Tetrahydro-5-nitro-pyrimidin-Derivate, ein Verfahren zu ihrer Herstellung und ihre Verwendung in Schädlingsbekämpfungsmitteln, insbesondere als Insektizide und Nematizide. Darüberhinaus besitzen die neuen Verbindungen eine stark ausgeprägte ektoparasitizide Wirksamkeit.

Es ist bereits bekannt, daß bestimmte Pyrimidino-thiazine wie z.B. 7-Ethyl-9-nitro-3,4,7,8-tetrahydro-(2H,6H)-pyrimidino-[4,3-b]-1,3-thiazin insektizide Eigenschaften aufweisen (vgl. US—PS—4 031 087).

Es wurden nun die neuen 1,2,3,6-Tetrahydro-5-nitro-pyrimidin-Derivate der Formel (I),

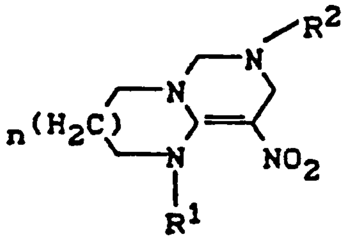

(I)

gefunden,
in welcher

n für die Zahlen 0, 1 oder 2 steht,

$R^1$ für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-triazolyl-$C_1$—$C_3$-alkyl, 1,2,3-Triazolyl-$C_1$—$C_3$-alkyl, Pyrimidinyl-$C_1$—$C_3$-alkyl, Pyrazinyl-$C_1$—$C_3$-alkyl, Pyridyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl und 1,3,4-Thiadiazolyl-$C_1$—$C_3$-alkyl steht und

$R^2$ für Dialkylamino mit jeweils 1 bis 6 Kohlenstoffatomen im Alkylteil; für Alkoxy mit 1 bis 6 Kohlenstoffatomen; für Alkenyloxy mit 3 bis 6 Kohlenstoffatomen; für Aralkoxy mit 6 bis 10 Kohlenstoffatomen im Arylteil und 1 bis 4 Kohlenstoffatomen im Alkylteil oder für gegebenenfalls durch Fluor, Chlor, Brom, Cyano, Nitro, Hydroxy, Mercapto, $C_1$—$C_4$-Alkoxy, $C_1$—$C_4$-Alkylthio, Halogen-$C_1$—$C_2$-alkoxy, Halogen-$C_1$—$C_2$-alkylthio, Amino, $C_1$—$C_4$-Alkylamino, Di-$C_1$—$C_4$-alkylamino, Hydroxycarbonyl, $C_1$—$C_4$-Alkoxycarbonyl, $C_1$—$C_4$-Alkylcarbonylamino, Morpholino oder $C_3$—$C_6$-Cycloalkyl substituiertes Alkyl mit 1 bis 20 Kohlenstoffatomen; für Alkenyl und Alkinyl mit jeweils 3 bis 6 Kohlenstoffatomen; für gegebenenfalls durch $C_1$—$C_2$-Alkyl, Fluor, Chlor, Brom oder Halogen-$C_1$—$C_2$-alkyl substituiertes Cycloalkyl mit 3 bis 8 Kohlenstoffatomen; für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-Triazolyl-$C_1$—$C_3$-alkyl, 1,2,3-Triazolyl-$C_1$—$C_3$-alkyl, Pyridyl-$C_1$—$C_3$-alkyl, Pyrazinyl-$C_1$—$C_3$-alkyl, Pyrimidinyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl, 1,3,4-Thiadiazolyl-$C_1$—$C_3$-alkyl steht und deren Säureadditions-Salze.

Weiterhin wurde gefunden, daß man die 1,2,3,6-Tetrahydro-5-nitro-pyrimidin-Derivate der Formel (I) und deren Säureadditions-Salze erhälte, wenn man Nitromethylen-Derivate der Formel (II)

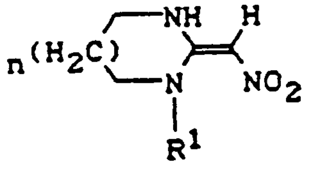

(II)

in welcher

$R^1$ und n die oben angegebenen Bedeutungen haben,
mit Aminen der Formel (III)

$$R^2NH_2 \qquad (III)$$

in welcher
$R^2$ die oben angegebenen Bedeutungen hat,
in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen physiologisch verträgliche Säuren addiert.

Überraschenderweise zeichnen sich die erfindungsgemäßen 1,2,3,6-Tetrahydro-5-nitro-pyrimidin-Derivate der Formel (I) in überragender Weise durch eine hohe Wirksamkeit als Insektizide, Nematizide und Ektoparasitizide aus.

Die Erfindung betrifft vorzugsweise Verbindungen der Formel (I), in welcher
n für die Zahlen 0 oder 1 steht,
$R^1$ für gegebenenfalls durch Fluor, Chlor, Brom, Cyano, Nitro, Methyl, Ethyl, Methoxy, Ethoxy, Methylthio, Ethylthio, Trifluormethyl, Trifluormethoxy oder Trifluormethylthio substituiertes Benzyl und Phenylethyl, sowie für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Thiophenylmethyl, Pyrazolylmethyl, 1,2,4-Triazolylmethyl, Pyrazinylmethyl, Pyrimidinylmethyl, Pyridylmethyl, Isoxazolylmethyl, Oxazolylmethyl, Thiazolylmethyl, 1,2,5-Thiadiazolylmethyl oder 1,3,4-Thiadiazolylmethyl steht und
$R^2$ für Dialkylamino mit jeweils 1 bis 4 Kohlenstoffatomen im Alkylteil; für Alkoxy mit 1 bis 4 Kohlenstoffatomen; 1 Prop-2-enyloxy; Phenylmethoxy, Phenylethoxy; für gegebenenfalls durch Fluor, Chlor, Brom, Cyano, Nitro, Hydroxy, Mercapto, Methoxy, Ethoxy, Methylthio, Ethylthio, Trifluormethoxy, Difluormethoxy, Trichlormethoxy, Chlordifluormethoxy, Trifluormethylthio, Difluormethylthio, Trichlormethylthio, Chlordifluormethylthio, Amino, Methylamino, Ethylamino, Dimethylamino, Diethylamino, Hydroxycarbonyl, $C_1$—$C_2$-Alkoxycarbonyl, $C_1$—$C_2$-Alkylcarbonylamino oder $C_3$—$C_6$-Cycloalkyl substituiertes Alkyl mit 1 bis 12 Kohlenstoffatomen; für Alkenyl und Alkinyl mit jeweils 3 oder 4 Kohlenstoffatomen; für gegebenenfalls durch Fluor, Chlor oder Trifluormethyl substituiertes Cycloalkyl mit 3 bis 6 Kohlenstoffatomen; für gegebenenfalls durch Fluor, Chlor, Brom, Cyano, Nitro, Methyl, Ethyl, Methoxy, Ethoxy, Methylthio, Ethylthio, Trifluormethyl, Trifluormethoxy oder Trifluormethylthio substituiertes Benzyl und Phenylethyl sowie für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Furylmethyl, Thiophenylmethyl, Pyrrollylmethyl, Pyrimidinylmethyl, Thiazolylmethyl, Pyrazolylmethyl, Morpholinomethyl oder Morpholino-n-propyl steht.

Bevorzugte erfindungsgemäße Verbindungen sind auch Additionsprodukte aus Säuren und denjenigen 1,2,3,6-Tetrahydro-5-nitro-pyrimidin-Derivaten der Formel (I), in denen die Substituenten R, $R^1$, $R^2$ oder der Index n die Bedeutungen haben, die bereits vorzugsweise für diese Substituenten und den Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Schwefelsäure, Salpetersäure, Essigsäure, Oxalsäure, Malonsäure, Bernsteinsäure, Äpfelsäure, Weinsäure, Maleinsäure, Fumarsäure, Methansulfonsäure, Benzoesäure, substituierte Benzoesäuren, Ameisensäure, Chloressigsäure, Toluolsulfonsäure, Benzolsulfonsäure, Trichloressigsäure, Phthalsäure, Naphthalinsulfonsäure, Nikotinsäure, Citronensäure und Ascorbinsäure.

Die beim erfindungsgemäßen Verfahren als Ausgangsstoffe zu verwendenden Nitromethylen-Derivate sind durch die Formel (II) allgemein definiert. In dieser Formel (II) stehen $R^1$ und n vorzugsweise für diejenigen Reste, die bereits im Zusammenhang mit der Beschreibung der erfindungsgemäßen Stoffe der formel (I) vorzugsweise für diesen Substituenten und den Index genannt wurden.

Die Verbindungen der Formel (II) sind bekannt und/oder lassen sich nach bekannten Methoden herstellen (vergl. z.B. DE—OS—2 514 402, EP—OS—136 636, EP—OS—154 178 und EP—OS—163 855).

Als Beispiele für die Verbindungen der Formel (II) seien genannt:

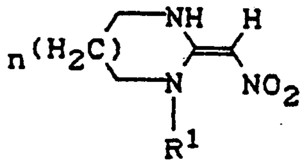

(II)

Tabelle 1

| n | $R^1$ | n | $R^1$ |
|---|---|---|---|
| 0 | 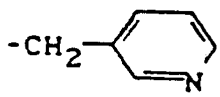 | 0 | 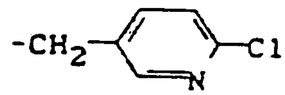 |
| 0 | 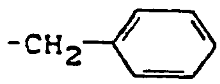 | 0 | 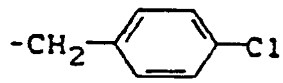 |
| 0 | 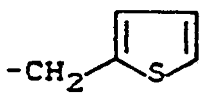 | 0 | 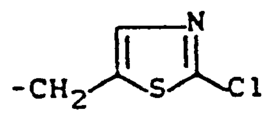 |
| 0 | 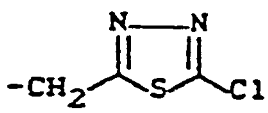 | 0 | 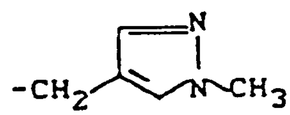 |
| 1 | 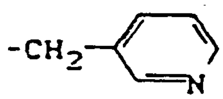 | 1 | 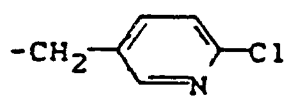 |
| 1 | 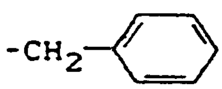 | 1 | 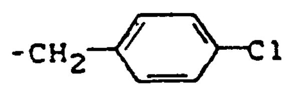 |
| 1 | 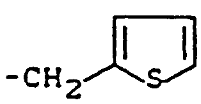 | 1 | 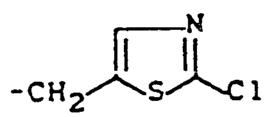 |
| 1 | 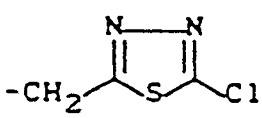 | 0 | 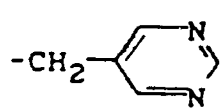 |
| 0 | 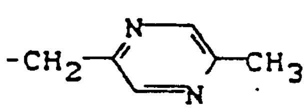 | | |
| 0 | 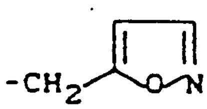 | | |

Die beim erfindungsgemäßen Verfahren außerdem als Ausgangsstoffe zu verwendenden Amine sind durch die Formel (III) allgemein definiert. In dieser Formel (III) steht $R^2$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Amine der Formel (III) sind allgemein bekannte Verbindungen der organischen Chemie.

Als Beispiele für die Verbindungen der Formel (III) seien genannt:

$$R^2NH_2 \qquad (III)$$

Tabelle 2

| $R^2$ | $R^2$ |
|---|---|
| $-CH_3$ | $-C_2H_5$ |
| $-C_3H_7$-n | $-C_3H_7$-i |
| $-C_4H_9$-n | $-C_4H_9$-i |
| $-C_4H_9$-sec. | $-C_4H_9$-tert. |
| 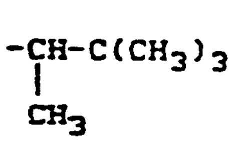 | 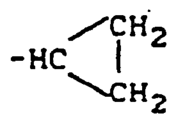 |
| 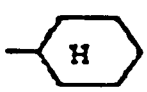 | $-N(CH_3)_2$ |
| $-N(C_2H_5)_2$ | $-N(C_3H_7\text{-}n)_2$ |

Tabelle 2 - Fortsetzung

| $R^2$ | $R^2$ |
|---|---|
| $-CH_2-CH=CH_2$ | $-CH_2-C\equiv CH$ |
| $-CH_2CH_2OH$ | $-CH_2CH_2N(CH_3)_2$ |
| $-CH_2CH_2N(C_2H_5)_2$ | $-CH_2CH_2N(C_3H_7\text{-}n)_2$ |
| 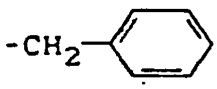 | 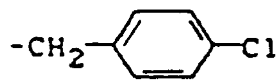 |
| 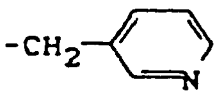 | 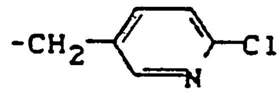 |
| 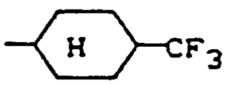 | 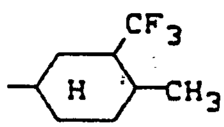 |
| 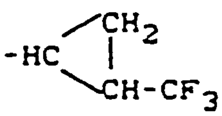 | 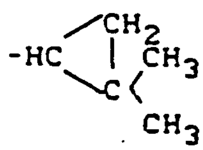 |
| 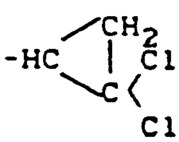 | 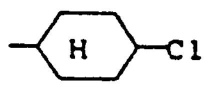 |
| $-CH_2CH_2CN$ | $-(CH_2)_5-CH_3$ |
| $-(CH_2)_4-CH_3$ | $-(CH_2)_{11}-CH_3$ |
| $-(CH_2)_{15}-CH_3$ | $-CH_2CH_2-OCH_3$ |
| $-CH_2CH_2CH_2OCH_3$ | $-(CH_2)_3O(CH_2)_3CH_3$ |
| 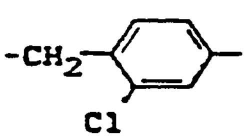 | 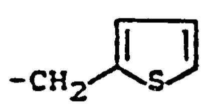 |

Tabelle 2 - Fortsetzung

| $R^2$ | $R^2$ |
|---|---|
| 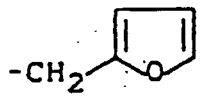 | 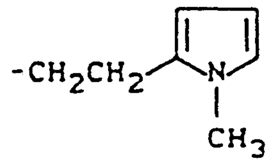 |
| $-CH_2-\overset{O}{\overset{\|}{C}}-OC_2H_5$ | 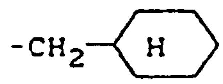 |
| 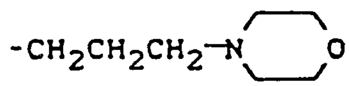 | $-CH_2CH_2CH_2OH$ |
| $-CH_2CH_2CH_2Cl$ | $-CH_2CH_2SH$ |
| $-CH_2CF_3$ | $-CH\langle{}^{CH_3}_{CF_3}$ |
| $-CH_2CH(OCH_3)_2$ | $-CH_2CH_2CO_2C_2H_5$ |
| $-CH_2CH_2NH\overset{O}{\overset{\|}{C}}CH_3$ | $-CH_2CO_2H$ |
| $-CH_2CH_2CO_2H$ | $-OCH_3$ |
| $-OC_2H_5$ | $-OCH_2CH=CH_2$ |
| 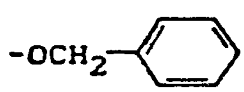 | |

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei Wasser und für die Umsetzung inerte organische Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl-, und Dibutylether, Glykoldimethylether und Diglykoldimethylether, Tetrahydrofuran und Dioxan, Alkohole, wie Methanol, Ethanol, n-Propanol und Isopropanol. Bevorzugt werden Gemische aus Alkoholen und Wasser eingesetzt.

Das erfindungsgemäße Verfahren wird gegebenenfalls in Gegenwart von sauren, nicht oxidierenden Katalysatoren durchgeführt. Besonders bewährt haben sich Halogenwasserstoffsäuren wie Salzsäure und Bromwasserstoffsäure, Phosphorsäure, niedere Carbonsäuren wie Essigsäure und Propionsäure.

Die Reaktionstemperaturen können bei dem erfindungsgemäßen Verfahren in einem größeren Bereich variiert werden. Im allgemeinen arbeitet man bei Temperaturen zwischen −20°C und +120°C, vorzugsweise bei Temperaturen zwischen 0°C und +80°C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Nitromethylen-Derivat der Formel (II), 1 bis 1,5 Mol, vorzugsweise 1 bis 1,2 Mol Amin der Formel (III) und 2 bis 4 Mol, vorzugsweise 2 bis 3 Mol Formaldehyd ein.

Die Amine der Formel (III) können gegebenenfalls als wäßrige Lösungen eingesetzt werden. Bei der Verwendung von gasförmigen Aminen der Formel (III) können diese Verbindungen durch das Gemisch aus Verdünnungsmittel, Verbindungen der Formel (II) und Formaldehyd geleitet werden. Für das erfindungsgemäße Verfahren wird Formaldehyd in wäßriger Lösung eingesetzt. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die Säureadditions-Salze der Verbindungen der Formel (I) können in einfacher Weise nach übichen Salzbildungsmethoden, z.B. durch Lösung einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltierten, isoliert und gegebenenfalls durch Waschen mit einem inerten organischen Lösungsmittel gereinigt werden.

Die Wirkstoffe eignen sich bei guter Pflanzenverträglichkeit und günstiger Warmblütertoxizität zur Bekämpfung von tierischen Schädlingen, insbesondere Insekten und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicornye brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus pinairius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa docemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Abthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichorodus spp.

Die erfindungsgemäßen Wirkstoffe der Formel (I) zeichnen sich durch eine hervorrangende insektizide und nematizide Wirksamkeit aus. Sie zeigen insbesondere beim Einsatz als Blattinsektizide und Bodeninsektizide eine hervorragende Wirkung gegen Maden wie z.B. Phorbia antiqua-Maden gegen Raupen, wie z.B. Plutella maculipennis, gegen Käferlarven, wie z.B. Phaedon cochleariae und Diabrotica balteata und Blattläuse, wie z.B. Myzus persicae und Aphis fabae. Sie zeigen außerdem eine sehr gute Wirksamkeit beim Einsatz gegen Nematoden, wie. z.B. Meloidogyne incognita.

Die neuen Verbindungen sind also besonders gut für einen Einsatz zur Bekämpfung von Blattinsekten, Bodeninsekten und Nematoden geeignet.

Weiterhin ziegen die neuen Verbindungen eine bakterizide Wirkung.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine

hervorragende Residualwirkung auf Holz und Ton sowie durch eine gute Alkalistabilität auf gekälkten Unterlagen aus.

Die erfindungsgemäßen Wirkstoff eignen sich auch zur Bekämpfung von Insekten, Milben, Zecken usw. auf dem Gebiet der Tierhaltung und Viehzucht, wobei durch die Bekämpfung der Schädlinge bessere Ergebnisse, z.B. höhere Milchleistungen, höheres Gewicht, schöneres Tierfell, längere Lebensdauer usw. erreicht werden können. Sie zeigen insbesondere beim Einsatz als Ektoparasitizide eine ausgezeichnete Wirkung gegen Blowfly-larven wie z.B. Lucilia cuprina.

Die Anwendung der erfindungsgemäßen Wirkstoffe geschieht auf diesem Gebiet in bekannter Weise, wie durch äußerliche Anwendung in Form beispielsweise des Tauchens (Dippen), Sprühens (Sprayen), Aufgießen (pour-on and spot-on) und des Einpuderns.

Die biologische Wirksamkeit der erfindungsgemäßen Verbindungen soll anhand der folgenden Beispiele erläutert werden.

## Herstellungsbeispiele

Beschreibung der Herstellungsmethodik:

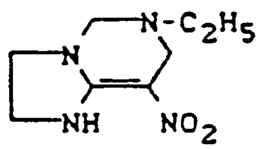

Zu einer Mischung aus 12,9 g (0,1 Mol) 2-Nitromethylenimidazolidin und 7,1 g (0,11 Mol) einer 70 %igen wäßrigen Lösung von Ethylamin in 50 ml Ethanol und 30 ml Wasser werden innerhalb von 1 Stunde bei 5°C—10°C 16,8 ml (0,22 Mol) 30 %ige wäßrige Formaldehydlösung zugetropft. Das Reaktionsgemisch wird anschließend 16 Stunden bei 25°C gerührt und das Lösungsmittel im Vakuum abdestilliert. Der Rückstand wird aus Essigester umkristallisiert.

Man erhält so 17,8 g (89% der Theorie) 6,7-Dihydro-6-ethyl-8-nitro-(5H)-imidazolidino-[2,3-f]-pyrimidin als beigefarbene Kristalle vom Schmelzpunkt 159°C.

Anolog dem erfindungsgemäßen Verfahren können die in der nachfolgenden Tabelle 3 angegebenen Verbindungen der Formel (I) hergestellt werden:

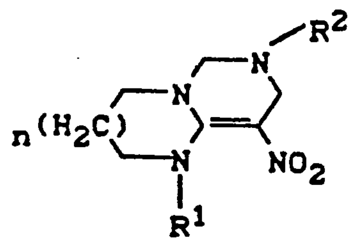

(I)

Tabelle 3

| Beisp.-Nr. | n | $R^1$ | $R^2$ | Physikal. Konstante |
|---|---|---|---|---|
| 1 | 0 | 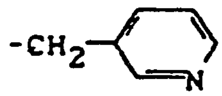 | $-C_3H_7$-i | Fp: 136-138° C |
| 2 | 0 | 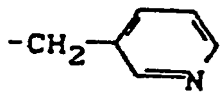 | $-C_2H_5$ | Fp: 142° C |
| 3 | 0 | 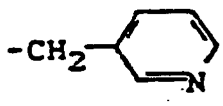 | $-CH_3$ | Fp: 189° C |
| 4 | 0 | 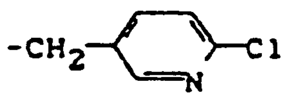 | $-C_3H_7$-i | Fp: 136-138° C |
| 5 | 0 | 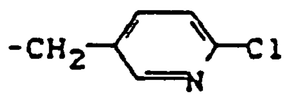 | $-CH_3$ | Fp: 150-152° C |
| 6 | 0 | 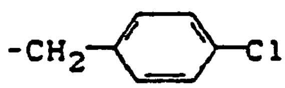 | $-CH_3$ | Fp: 156-158° C |
| 7 | 0 | 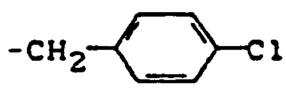 | $-C_2H_5$ | Fp: 180-182° C |
| 8 | 0 | 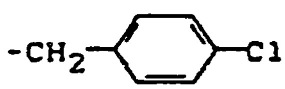 | $-C_3H_7$-i | Fp: 150-151° C |
| 9 | 0 | 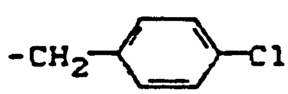 | $-C_3H_7$-n | Fp: 166° C |
| 10 | 1 | 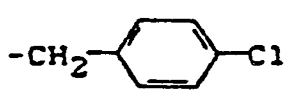 | $-CH_3$ | Fp: 168-170° C |

Tabelle 3 - Fortsetzung.

| Beisp.-Nr. | n | $R^1$ | $R^2$ | Physikal. Konstante |
|---|---|---|---|---|
| 11 | 1 | $-CH_2$- 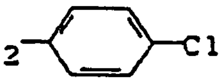 | $-C_2H_5$ | Fp:156-158° C |
| 12 | 1 | $-CH_2$- 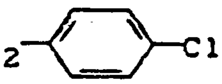 | $-C_3H_7$-i | Fp:140° C |
| 13 | 1 | $-CH_2$- 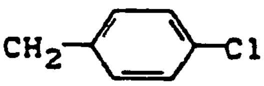 | $-C_3H_7$-n | Fp:138-140° C |
| 14 | 1 | $-CH_2$- 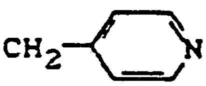 | $-CH_3$ | Fp:136° C |
| 15 | 1 | $-CH_2$- 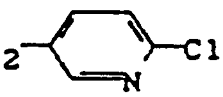 | $-CH_3$ | Fp:142° C |
| 16 | 0 | $-CH_2$- 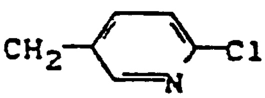 | $-C_2H_5$ | Fp:178° C |
| 17 | 0 | $-CH_2$- 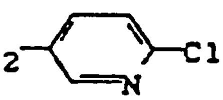 | $-C_4H_9$-tert. | Fp:128° C |
| 18 | 0 | $-CH_2$- 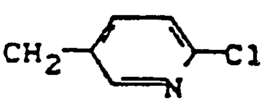 | $-CH_2CH_2OH$ | Fp:160-162° C |
| 19 | 0 | $-CH_2$- 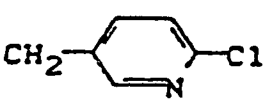 | 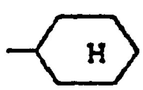 | Fp:168° C |
| 20 | 0 | $-CH_2$- 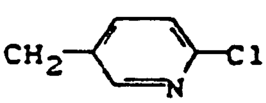 | $-CH_2$- 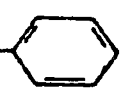 | Fp:150-152° C |

Tabelle 3 - Fortsetzung

| Beisp.-Nr. | n | $R^1$ | $R^2$ | Physikal. Konstante |
|---|---|---|---|---|
| 21 | 0 | 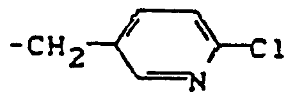 | 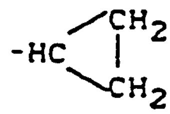 | Fp: 150° C |
| 22 | 1 | 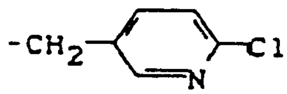 | $-C_2H_5$ | Fp: 144° C |
| 23 | 1 | 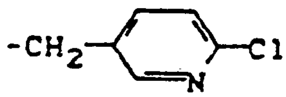 | $-C_3H_7$-i | Fp: 142° C |
| 24 | 1 | 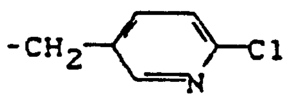 | 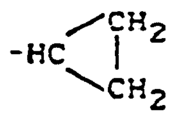 | Fp: 82° C |
| 25 | 0 | 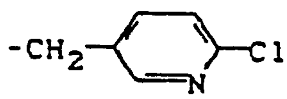 | $-CH_2-CH=CH_2$ | Fp: 144° C |
| 26 | 0 | 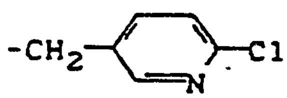 | $-C_4H_9$-n | Fp: 134° C |
| 27 | 0 | 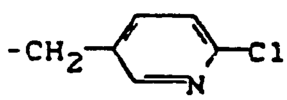 | $-C_6H_{13}$-n | Fp: 134° C |

Tabelle 3 - Fortsetzung

| Beisp.-Nr. | n | $R^1$ | $R^2$ | Physikal. Konstante |
|---|---|---|---|---|
| 28 | 0 | $-CH_2-$ 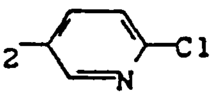 | $-CH_2CH_2OCH_3$ | Fp: 169° C |
| 29 | 0 | $-CH_2-$ 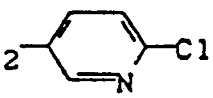 | $-CH_2-$ 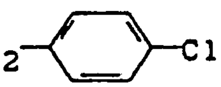 | Fp: 186° C |
| 30 | 0 | $-CH_2-$ 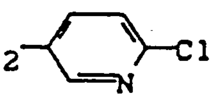 | $-CH_2-$ 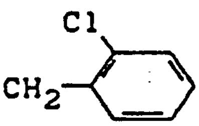 | Fp: 94° C |
| 31 | 0 | $-CH_2-$ 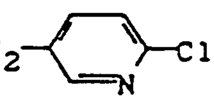 | $-C_{12}H_{25}-n$ | Fp: 117° C |
| 32 | 0 | $-CH_2-$ 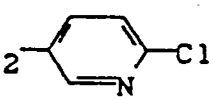 | $-C_5H_{11}-n$ | Fp: 129° C |
| 33 | 0 | $-CH_2-$ 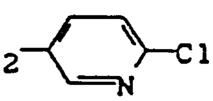 | $-C_{16}H_{33}-n$ | Fp: 122° C |
| 34 | 0 | $-CH_2-$ 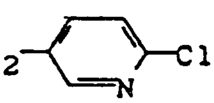 | $-(CH_2)_3-O-(CH_2)_3CH_3$ | Fp: 78° C |
| 35 | 0 | $-CH_2-$ 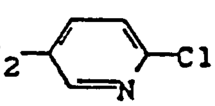 | $-CH_2CH_2-N(C_2H_5)_2$ | Fp: 125° C |
| 36 | 0 | $-CH_2-$ 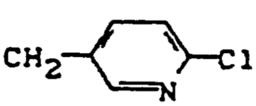 | $-CH_2-$ 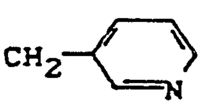 | Fp: 134° C |
| 37 | 0 | $-CH_2-$ 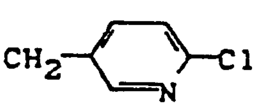 | $-CH_2CH_2CH_2-$ 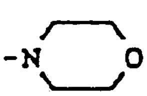 | Fp: 120° C |

Tabelle 3 - Fortsetzung

| Beisp.- Nr. | n | $R^1$ | $R^2$ | Physikal. Konstants |
|---|---|---|---|---|
| 38 | 0 | 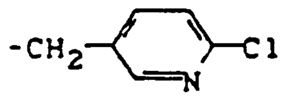 | 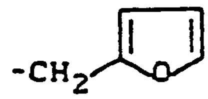 | Fp:160° C |
| 39 | 0 | 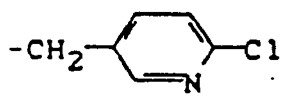 | 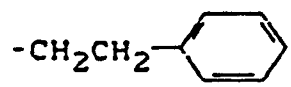 | Fp:114° C |
| 40 | 0 | 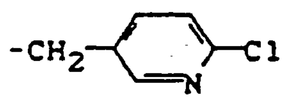 | $-CH_2CH(OCH_3)_2$ | Fp:150° C |
| 41 | 0 | 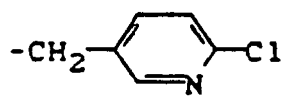 | 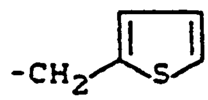 | Fp:158° C |
| 42 | 0 | 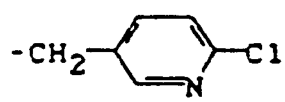 | $-CH_2CH_2COOC_2H_5$ | |
| 43 | 0 | 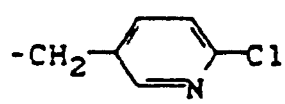 | $-CH_2CH_2CH_2Cl$ | Fp:120° C |
| 44 | 0 | 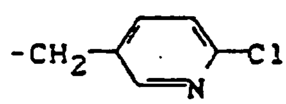 | $-CH_2CH_2SH$ | |
| 45 | 0 | 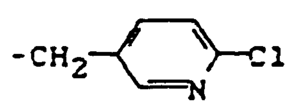 | $-N(CH_3)_2$ | |
| 46 | 0 | 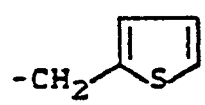 | $-CH_3$ | Fp:112° C |
| 47 | 1 | 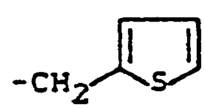 | $-CH_3$ | |

Tabelle 3 - Fortsetzung

| Beisp.-Nr. | n | $R^1$ | $R^2$ | Physikal. Konstante |
|---|---|---|---|---|
| 48 | 0 | 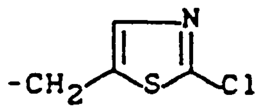 | $-CH_3$ | *δ=4,95(s) |
| 49 | 1 | 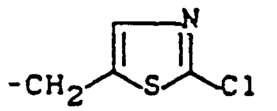 | $-CH_3$ | *δ=4,66(s) |
| 50 | 0 | 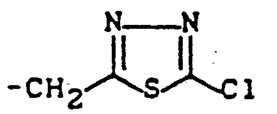 | $-CH_3$ | |
| 51 | 1 | 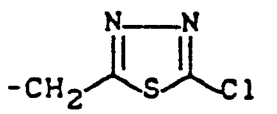 | $-CH_3$ | |
| 52 | 0 | 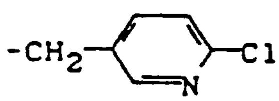 | 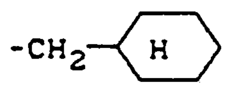 | Fp:150° C |
| 53 | 0 | 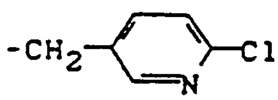 | $-CH_2COOC_2H_5$ | Fp:147° C |
| 54 | 0 | 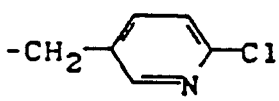 | $-CH_2CH_2NH-COCH_3$ | |
| 55 | 0 | 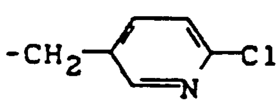 | $-CH_2COOH$ | Fp:88° C |
| 56 | 0 | 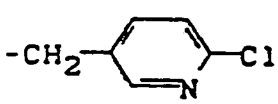 | $-CH_2CH_2COOH$ | |
| 57 | 0 | 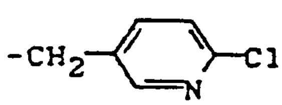 | $-OCH_3$ | |

Tabelle 3 - Fortsetzung

| Beisp.-Nr. | n | $R^1$ | $R^2$ | Physikal. Konstante |
|---|---|---|---|---|
| 58 | 0 | 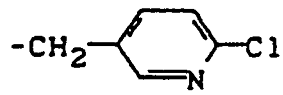 | $-OC_2H_5$ | |
| 59 | 0 | 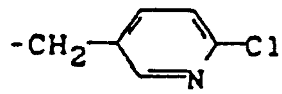 | $-OCH_2CH=CH_2$ | |
| 60 | 0 | 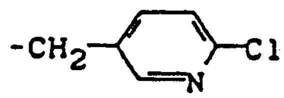 | 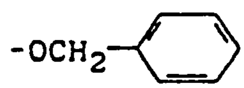 | |
| 61 | 0 | 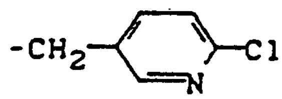 | $-CH_2CF_3$ | Fp:143° C |
| 62 | 0 | 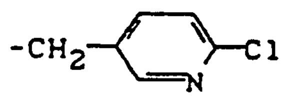 | 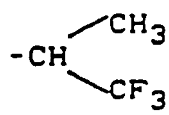 | Fp:163° C |
| 63 | 1 | 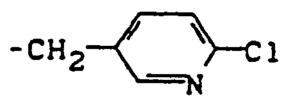 | $-CH_2C{\equiv}CH$ | *δ=4,60 (s) |
| 64 | 0 | 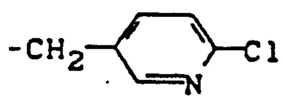 | $-CH_2CH_2CH_2OCH_3$ | Fp:181° C |
| 65 | 0 | 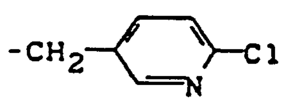 | $-CH_2CH_2CH_2OH$ | Fp:>230° C |
| 66 | 0 | 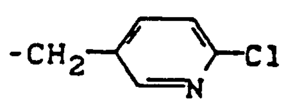 | 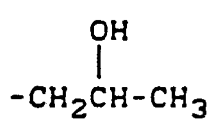 | Fp:146° C |
| 67 | 0 | 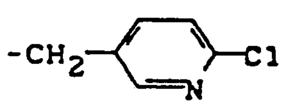 | $-CH_2CH_2CN$ | Fp: 87° C |
| 68 | 0 | 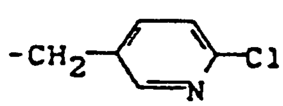 | $-C(CH_3)_2CH_2CH_3$ | Fp:126° C |

Tabelle 3 - Fortsetzung

| Beisp.-Nr. | n | $R^1$ | $R^2$ | Physikal. Konstante |
|---|---|---|---|---|
| 69 | 0 | 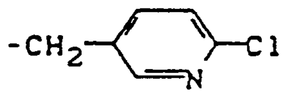 | 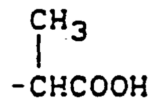 | *δ=4,74(q) |
| 70 | 0 | 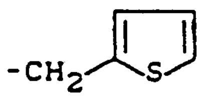 | $-C(CH_3)_2CH_2CH_3$ | *δ=4,98(s) |
| 71 | 0 | 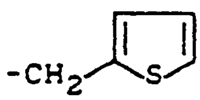 | $-CH_2CH=CH_2$ | Fp:104° C |
| 72 | 0 | 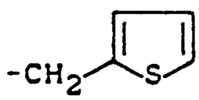 | 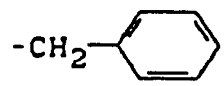 | Fp:164° C |
| 73 | 0 | 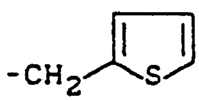 | $-C_2H_5$ | Fp:177° C |
| 74 | 0 | 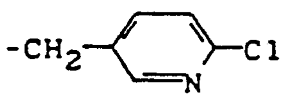 | $-C(CH_2F)_2CH_3$ | Fp:164° C |
| 75 | 0 | 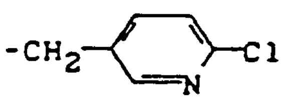 | 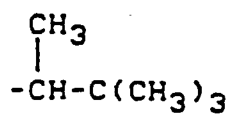 | Fp:156° C |
| 76 | 0 | 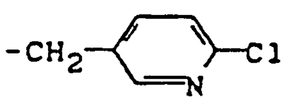 | $-CH_2C(CH_3)_3$ | Fp:150° C |
| 77 | 0 | 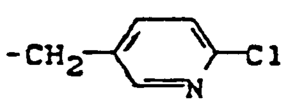 | 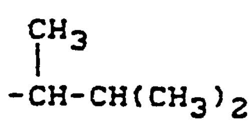 | Fp:122° C |

Tabelle 3 - Fortsetzung

| Beisp.-Nr. | n | $R^1$ | $R^2$ | Physikal. Konstante |
|---|---|---|---|---|
| 78 | 0 | -$CH_2$- 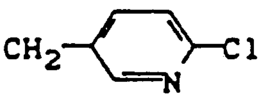 | 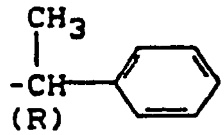 (R) | Fp: 131° C |
| 79 | 0 | -$CH_2$- 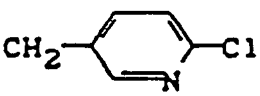 | 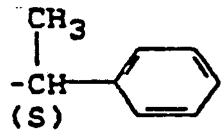 (S) | Fp: 132° C |
| 80 | 0 | -$CH_2$- 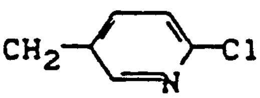 | 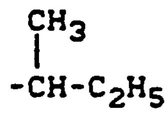 | Fp: 152° C |
| 81 | 0 | -$CH_2$- 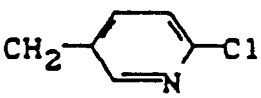 | $-CH_2CH(CH_3)_2$ | Fp: 138° C |
| 82 | 0 | -$CH_2$- 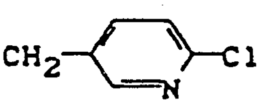 | $-C_3H_7$-n | Fp: 172° C |
| 83 | 0 | -$CH_2$- 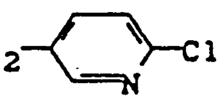 | 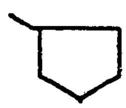 | Fp: 131° C |
| 84 | 0 | -$CH_2$- 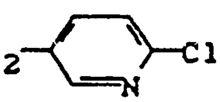 | 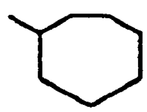 | Fp: 115° C |
| 85 | 0 | -$CH_2$- 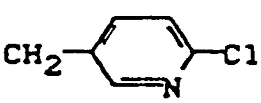 | $-CH_2CH_2OC_2H_5$ | Fp: 146° C |
| 86 | 0 | -$CH_2$- 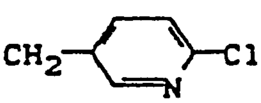 | 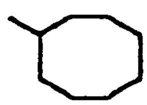 | Fp: 134° C |

Tabelle 3 - Fortsetzung

| Beisp.-Nr. | n | $R^1$ | $R^2$ | Physikal. Konstante |
|---|---|---|---|---|
| 87 | 0 | 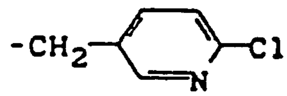 | 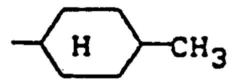 | Fp: 110° C |
| 88 | 0 | 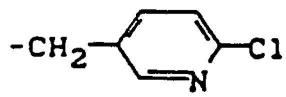 | 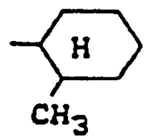 | Fp: 148° C |
| 89 | 0 | 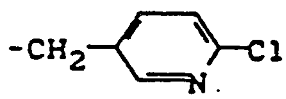 | 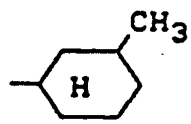 | Fp: 133° C |
| 90 | 0 | 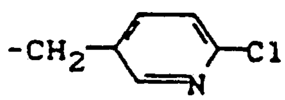 | 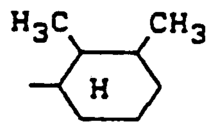 | Fp: 134° C |
| 91 | 0 | 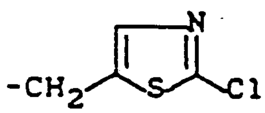 | $-C(CH_3)_2CH_2CH_3$ | Fp: 127° C |
| 92 | 1 | 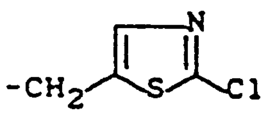 | $-C(CH_3)_2CH_2CH_3$ | Fp: 149° C |
| 93 | 0 | 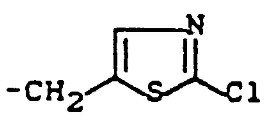 | 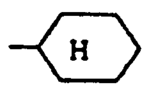 | *δ=4,92(s) |
| 94 | 0 | 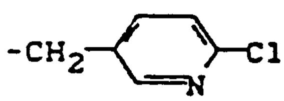 | 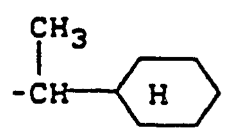 | Fp: 133° C |
| 95 | 0 | 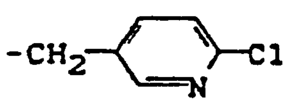 | 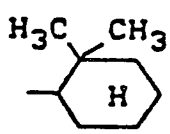 | Fp: 142° C |

Tabelle 3 - Fortsetzung

| Beisp.-Nr. | n | $R^1$ | $R^2$ | Physikal. Konstante |
|---|---|---|---|---|
| 96 | 0 | 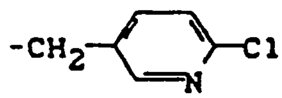 | 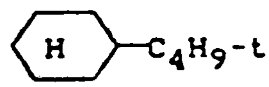 | Fp:172° C |
| 97 | 0 | 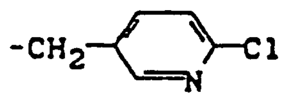 | 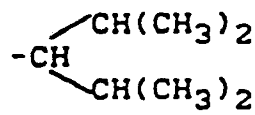 | Fp:114° C |
| 98 | 0 | 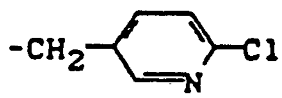 | 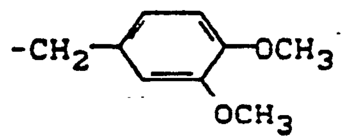 | Fp:115° C |
| 99 | 0 | 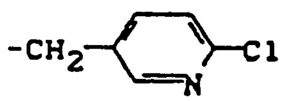 | $-CH_2CH_2SC_2H_5$ | Fp:180° C |
| 100 | 0 | 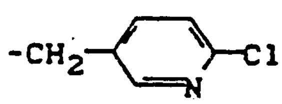 | $-(CH_2)_6CH_3$ | Fp:147° C |
| 101 | 0 | 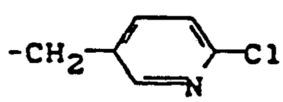 | $-(CH_2)_7CH_3$ | Fp:147° C |
| 102 | 0 | 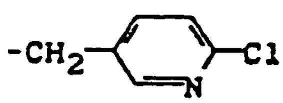 | $-(CH_2)_8CH_3$ | Fp:129° C |
| 103 | 0 | 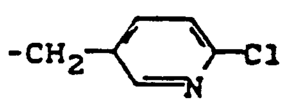 | $-(CH_2)_9CH_3$ | Fp:117° C |
| 104 | 0 | 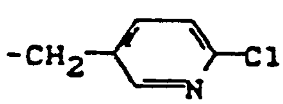 | $-(CH_2)_{10}CH_3$ | Fp:115° C |
| 105 | 0 | 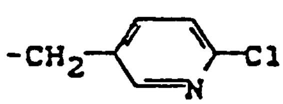 | $-(CH_2)_{13}CH_3$ | Fp:120° C |

Tabelle 3 - Fortsetzung

| Beisp.-Nr. | n | $R^1$ | $R^2$ | Physikal. Konstante |
|---|---|---|---|---|
| 106 | 0 | 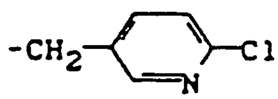 | $-CH_2-CH(C_2H_5)-(CH_2)_3CH_3$ | Fp: 119° C |
| 107 | 0 | 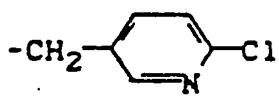 | $-(CH_2)_{17}CH_3$ | Fp: 120° C |
| 108 | 0 | 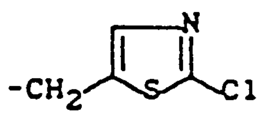 | $-CH(CH_3)-C(CH_3)_3$ | Fp: 142° C |
| 109 | 0 | 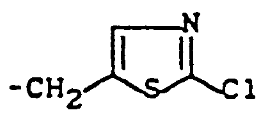 | $-CH_2CH(OCH_3)_2$ | Fp: 124° C |
| 110 | 1 | 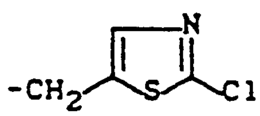 | $-CH(CH_3)-C(CH_3)_3$ | Fp: 144° C |
| 111 | 1 | 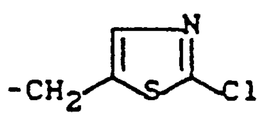 | $-(CH_2)_{11}CH_3$ | Fp: 103° C |
| 112 | 1 | 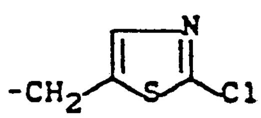 | $-(CH_2)_5CH_3$ | Fp: 76° C |
| 113 | 1 | 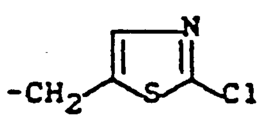 | $-(CH_2)_6CH_3$ | Fp: 89° C |
| 114 | 1 | 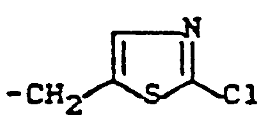 | $-(CH_2)_7CH_3$ | Fp: 79° C |
| 115 | 1 | 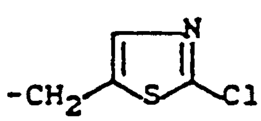 | $-(CH_2)_8CH_3$ | Fp: 70° C |

Tabelle 3 - Fortsetzung

| Beisp.-Nr. | n | $R^1$ | $R^2$ | Physikal. Konstante |
|---|---|---|---|---|
| 116 | 1 | 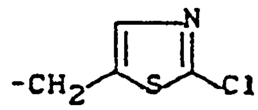 | $-(CH_2)_9CH_3$ | Fp: 89° C |
| 117 | 0 | 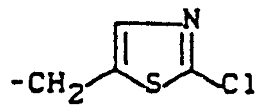 | $-(CH_2)_5CH_3$ | Fp: 75° C |
| 118 | 0 | 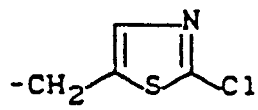 | $-(CH_2)_{11}CH_3$ | Fp: 81° C |
| 119 | 0 | 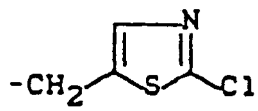 | $-(CH_2)_{17}CH_3$ | Fp: 89° C |
| 120 | 0 | 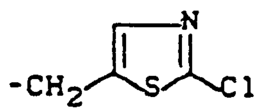 | $-(CH_2)_{10}CH_3$ | Fp:104° C |
| 121 | 0 | 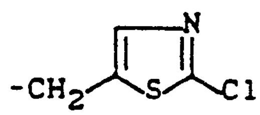 | $-(CH_2)_6CH_3$ | Fp:114° C |
| 122 | 0 | 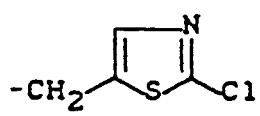 | $-(CH_2)_{13}CH_3$ | Fp:111° C |
| 123 | 0 | 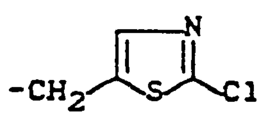 | $-(CH_2)_{15}CH_3$ | Fp:111° C |
| 124 | 0 | 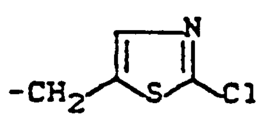 | $-(CH_2)_7CH_3$ | Fp:120° C |
| 125 | 0 | 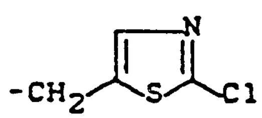 | $-(CH_2)_8CH_3$ | Fp:111° C |
| 126 | 0 | 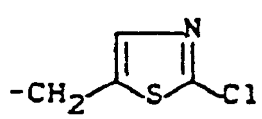 | $-(CH_2)_9CH_3$ | Fp:103° C |

Tabelle 3 - Fortsetzung

| Beisp.-Nr. | n | $R^1$ | $R^2$ | Physikal. Konstante |
|---|---|---|---|---|
| 127 | 0 | 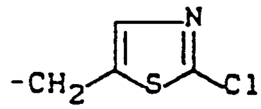 | $-(CH_2)_8CH{=}CH(CH_2)_7CH_3$ | Fp:82° C |
| 128 | 0 | 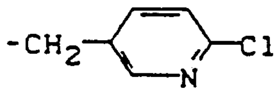 | $-(CH_2CH_2O)_4H$ | Fp:101° C |
| 129 | 0 | 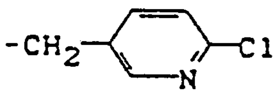 | $-(CH_2CH_2O)_2CH_3$ | Fp:118° C |
| 130 | 0 | 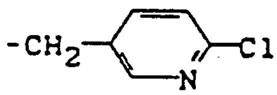 | $-CH_2-CH_2OCH_2CH_2-N(CH_3)_2$ | Fp: 115° C |
| 131 | 0 | 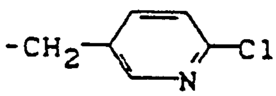 | $-(CH_2CH_2O)_2C_4H_9$ | Fp:118° C |
| 132 | 0 | 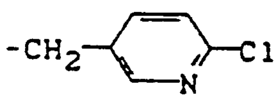 | $-(CH_2CH_2O)_2C_2H_5$ | Fp:160° C |
| 133 | 0 | 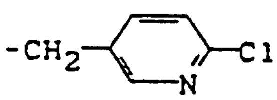 | $-(CH_2CH_2O)_3H$ | Fp:135° C |
| 134 | 0 | 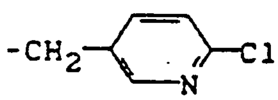 | $-(CH_2CH_2O)_3CH_3$ | Fp:110° C |
| 135 | 0 | 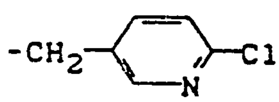 | $-(CH_2CH_2O)_2H$ | Fp:140° C |
| 136 | 0 | 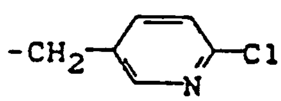 | $-(CH_2)_8CH{=}CH(CH_2)_7CH_3$ | Fp: 95° C |

* Sind die δ-Werte der $^1$H-NMR-Spektren, die in $CDCl_3$ aufgenommen wurden. Angegeben sind die chemischen Verschiebungen (chemical Shifts) für die Gruppierung

$$-CH_2-\overset{NO_2}{\overset{|}{C}}= \;.$$

Verwendungsbeispiele

In den folgenden Verwendungsbeispielen wurde die folgende Verbindung als Vergleichssubstanz eingesetzt:

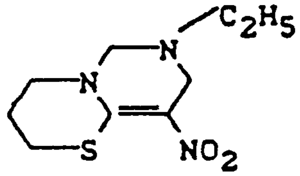

(A)

7-Ethyl-9-nitro-3,4,7,8-tetrahydro-(2H, 6H)-pyrimidino-[4,3-b]-1,3-thiazin aus US—PS—4 031 087.

Beispiel A

Phaedon-Larven-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käferlarven abgetötet wurden; 0% bedeutet, daß keine Käfer-Larven abgetötet wurden.

Bei diesem Test zeigen z.B. die folgenden Verbindungen der Herstellungsbeispiele (2), (3), (4), (5), (15), (16), (17), (18), (20), (21), (22), (23), (24), (25), (26), (27), (28), (29), (31) und (32) bei einer Wirkstoffkonzentration von 0,01% nach 3 Tagen eine Wirkung von 100%, während die Vergleichssubstanz (A) keine Wirkung zeigt.

Beispiel B

Plutella-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele (1), (2), (4), (5), (16), (17), (18), (19), (20), (21) und (25) bei einer Wirkstoffkonzentration von 0,01% nach 3 Tagen eine Wirkung von 100%, während die Vergleichssubstanz (A) keine Wirkung zeigt.

Beispiel C

Myzus-Test

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallenb sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z.B. die folgenden Verbindungen der Herstellungsbeispiele (3), (4), (5), (16), (17), (18), (20), (21), (22), (23), (24), und (25) bei einer Wirkstoffkonzentration von 0,01% nach 1 Tag eine Wirkung von 99—100%, während die Vergleichssubstanz (A) keine Wirkung von 40% zeigt.

Beispiel D

Aphis-Test (systemische Wirkung)

Lösungsmittel: 7 Gewichtsteile Dimethylformamid

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Mit je 20 ml Wirkstoffzubereitung der gewünschten Konzentration werden Bohnenpflanzen (Vicia fabs), die stark von der schwarzen Bohnenlaus (Aphis fabae) befallen wird, so daß dis Wirkstoffzubereitung in den Boden endringt, ohne den Sproß zu benetzen. Der Wirkstoff wird von den Wurzeln aufgenommen und in den Sproß weitergeleitet.

Nach der gewünschten Zeit wird die Abtötung in % bestimmt. Dabei bedeutet 100%, daß alle Blattläuse abgetötet wurden; 0% bedeutet, daß keine Blattläuse abgetötet wurden.

Bei diesem Test zeigen z.B. die folgenden Verbindungen der Herstellungsbeispiele (2), (3), (4), (5), (15), (16), (17), (18), (19), (20), (21), (22), (24), (25), (26), (27), (28), (31) und (32) bei einer Wirkstoffkonzentration von 0,01% nach 4 Tagen eine Wirkung von 90 bis 100%, während die Vergleichssubstanz (A) keine Wirkung zeigt.

Beispiel E

Grenzkonzentrations-Test/Bodeninsekten

Testinsekt: Phorbia antiqua-Maden im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegebenen wird. Man füllt den Boden in Töpfe und läßt diese bei Raumtemperatur stehen.

Nach 24 Stunden werden die Testtiere in den benhandelten Boden gegeben und nach weiteren 2 bis 7 Tagen wird der Wirkungsrad des Wirkstoffs durch Auszählen der toten und lebenden Testinsekten in % bestimmt. Der Wirkungsrad ist 100% wenn alle Testinsekten abgetötet worden sind, er ist 0%, wenn noch genau so viele Testinsekten leben wie bei der unbehandelten Kontrolle.

Bei diesem Test zeigen z.B. die Verbindungen der Herstellungsbeispiele (5) bei einer Wirkstoffkonzentration von 20 ppm eine Wirkung von 100%, während die Vergleichssubstanz (A) keine Wirkung zeigt.

Beispiel F

Grenzkonzentrations-Test/Bodeninsekten

Testinsekt: Diabrotica balteata-Larven im Boden

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegebenen wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsrad des Wirkstoffs durch Auszählen der toten und lebenden Testinsekten in % bestimmt. Der Wirkungsrad ist 100%, wenn alle Testinsekten abgetötet worden sind, er ist 0%, wenn noch genau so viele Testinsekten leben wie bei der unbehandelten Kontrolle.

Bei diesem Test zeigen z.B. die Verbindungen der Herstellungsbeispiele (5) bei einer Wirkstoffkonzentration von 20 ppm eine Wirkung von 100%, während die Vergleichssubstanz (A) keine Wirkung zeigt.

Beispiel G

Grenzkonzentrations-Test/Wurzelsystemische Wirkung

Testinsekt: Phaedon cochleariae-Larven

Lösungsmittel: 3 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit

der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegebenen wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiet werden.

Für den Nachweise des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. As den Abtötungszahln wird die wurzelsystemische Wirkung des Wirkstoffs abgeleitet. Sie ist 100%, wenn alle Testiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele (5), (38), (41), (62) und (77) bei einer Wirkstoffkonzentration von 20 ppm eine Wirkung von 100%, während die Vergleichssubstanz (A) keine Wirkung zeigt.

Beispiel H

Grenzkonzentrations-Test/Wurzelsystemische Wirkung
Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegebenen wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiet werden.

Für den Nachweise des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die Wurzelsystemische Wirkung des Wirkstoffs abgeleitet. Sie ist 100%, wenn alle Testiere abgetötet sind und 0%, wenn noch genau so viele Testinsekten leben wie bei der unbehandelten Kontrolle.

Bei diesem Test zeigen z.B. die folgenden Verbindungen der Herstellungsbeispiele (1), (2), (3), (5), (18), (31), (38) , (41), (62) und (77) bei einer Wirkstoffkonzentration vom 20 ppm eine Wirkung von 100%, während die Vergleichssubstanz (A) keine Wirkung zeigt.

Beispiel I

Grenzkonzentrations-Test
Testnematode: Meloidogyne incognita
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18°C.

Nach ses Wochen werden die Kartoffelwurzeln auf Zysten untersuch und er Wirkungsgrad des Wirkstoffs in % bestimmt. Der Wirkungsrad ist 100%, wenn der Befall vollständig vermieden wird, er ist 0%, wenn der Befall genau so hoch ist wei bei den Kontrollpflanzen in unbehandeltem, aber in gleicher Weise verseuchtem Boden.

Bei diesem Test zeigen z.B. die folgenden Verbindungen der Herstellungsbeispiele (14) und (15) bei einer Wirkstoffkonzentration von 20 ppm eine Wirkung von 100%, während die Vergleichssubstanz (A) keine Wirkung zeigt.

Beispiel J

Test mit Lucilia cuprina resistent-Larven
Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteil Wirkstoff

mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in sein Teströhrchen gebracht, welches ca. 1 $cm^3$ Pferdefleisch und 0,5 ml de Wirkstoffzubereitung enthalt. Nach 24 Stunden wird der Abtötungsgrad bestimmt.

Bei diesem Test zeigen z.B. die folgenden Verbindungen der Herstellungsbeispiele (1), (2), (3), (5), (6), (8), (10), (12) (13), (15), (19), (20), (21), (22), (23), (24) und (25) bei einer Wirkstoffkonzentration von 1000 ppm eine Abtötung von 100%.

**Patentansprüche**

1. 1,2,3,6-Tetrahydro-5-nitro-pyrimidin-Derivate der Formel (I)

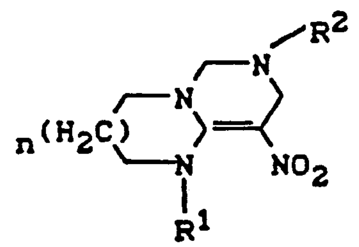

(I)

in welcher

n für die Zahlen 0, 1 oder 2 steht,

$R^1$ für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-Triazolyl-$C_1$—$C_3$-alkyl, 1,2,3-Triazolyl-$C_1$—$C_3$-alkyl, Pyrimidinyl-$C_1$—$C_3$-alkyl, Pyrazinyl-$C_1$—$C_3$-alkyl, Pyridyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl und 1,3,4-Thiadiazolyl-$C_1$—$C_3$-alkyl steht und

$R^2$ für Dialkylamino mit jeweils 1 bis 6 Kohlenstoffatomen im Alkylteil; für Alkoxy mit 1 bis 6 Kohlenstoffatomen; für Alkenyloxy mit 3 bis 6 Kohlenstoffatomen; für Aralkoxy mit 6 bis 10 Kohlenstoffatomen im Arylteil und 1 bis 4 Kohlenstoffatomen im Alkylteil oder für gegebenenfalls durch Fluor, Chlor, Brom, Cyano, Nitro, Hydroxy, Mercapto, $C_1$—$C_4$-Alkoxy, $C_1$—$C_4$-Alkylthio, Halogen-$C_1$—$C_2$-alkoxy, Halogen-$C_1$—$C_2$-alkylthio, Amino, $C_1$—$C_4$-Alkylamino, Di-$C_1$—$C_4$-alkylamino, Hydroxycarbonyl, $C_1$—$C_4$-Alkoxycarbonyl, $C_1$—$C_4$-Alkylcarbonylamino, Morpholino oder $C_3$—$C_6$-Cycloalkyl substituiertes Alkyl mit 1 bis 20 Kohlenstoffatomen; für Alkenyl und Alkinyl mit jeweils 3 bis 6 Kohlenstoffatomen; für gegebenenfalls durch $C_1$—$C_2$-Alkyl, Fluor, Chlor, Brom oder Halogen-$C_1$—$C_2$-alkyl substituiertes Cycloalkyl mit 3 bis 8 Kohlenstoffatomen; für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-Triazolyl-$C_1$—$C_3$-alkyl, 1,2,3-Triazolyl-$C_1$—$C_3$-alkyl, Pyridyl-$C_1$—$C_3$-alkyl, Pyrazinyl-$C_1$—$C_3$-alkyl, Pyrimidinyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl, 1,3,4-Thiadiazolyl-$C_1$—$C_3$-alkyl steht und deren Säureadditionssalze.

2. 1,2,3,6-Tetrahydro-5-nitro-pyrimidin-Derivate der Formel (I) gemäß Anspruch 1, in welcher

n für die Zahlen 0 oder 1 steht,

$R^1$ für gegebenenfalls durch Fluor, Chlor, Brom, Cyano, Nitro, Methyl, Ethyl, Methoxy, Ethoxy, Methylthio, Ethylthio, Trifluormethyl, Trifluormethoxy oder Trifluormethylthio substituiertes Benzyl und Phenylethyl, sowie für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Thiophenylmethyl, Pyrazolylmethyl, 1,2,4-Triazolylmethyl, Pyrazinylmethyl, Pyrimidinylmethyl, Pyridylmethyl, Isoxazolylmethyl, Oxazolylmethyl, Thiazolylmethyl, 1,2,5-Thiadiazolylmethyl oder 1,3,4-Thiadiazolylmethyl steht und

$R^2$ für Dialkylamino mit jeweils 1 bis 4 Kohlenstoffatomen im Alkylteil; für Alkoxy mit 1 bis 4

Kohlenstoffatomen; 1 Prop-2-enyloxy; Phenylmethoxy, Phenylethoxy; für gegebenenfalls durch Fluor, Chlor, Brom, Cyano, Nitro, Hydroxy, Mercapto, Methoxy, Ethoxy, Methylthio, Ethylthio, Trifluormethoxy, Difluormethoxy, Trichlormethoxy, Chlordifluormethoxy, Trifluormethylthio, Difluormethylthio, Trichlormethylthio, Chlordifluormethylthio, Amino, Methylamino, Ethylamino, Dimethylamino, Diethylamino, Hydroxycarbonyl, $C_1$—$C_2$-Alkoxycarbonyl, $C_1$—$C_2$-Alkylcarbonylamino oder $C_3$—$C_6$-Cycloalkyl substituiertes Alkyl mit 1 bis 12 Kohlenstoffatomen; für Alkenyl und Alkinyl mit jeweils 3 oder 4 Kohlenstoffatomen; für gegebenenfalls durch Fluor, Chlor oder Trifluormethyl substituiertes Cycloalkyl mit 3 bis 6 Kohlenstoffatomen; für gegebenenfalls durch Fluor, Chlor, Brom, Cyano, Nitro, Methyl, Ethyl, Methoxy, Ethoxy, Methylthio, Ethylthio, Trifluormethyl, Trifluormethoxy oder Trifluormethylthio substituiertes Benzyl und Phenylethyl sowie für gegebenenfalls durch Fluor, Chlor, Brom, Cyano, Nitro, Methyl, Ethyl, Methoxy, Methylthio, Trifluormethyl, Trifluormethoxy, Trifluormethylthio, Dimethylamino oder Diethylamino substituiertes Furylmethyl, Thiophenylmethyl, Pyrrollylmethyl, Pyrimidinylmethyl, Thiazolylmethyl, Pyrazolylmethyl, Morpholinomethyl oder Morpholino-n-propyl steht.

3. Verfahren zur Herstellung von 1,2,3,6-Tetrahydro-5-nitro-pyrimidin-Derivaten der Formel (I)

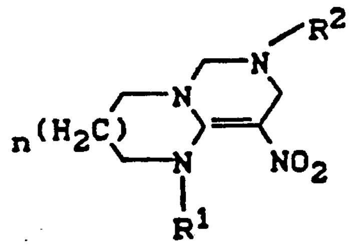

(I)

in welcher

n für die Zahlen 0, 1 oder 2 steht,

$R^1$ für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-Triazolyl-$C_1$—$C_3$-alkyl, 1,2,3-Triazolyl-$C_1$—$C_3$-alkyl, Pyrimidinyl-$C_1$—$C_3$-alkyl, Pyrazinyl-$C_1$—$C_3$-alkyl, Pyridyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl und 1,3,4-Thiadiazolyl-$C_1$—$C_3$-alkyl steht und

$R^2$ für Dialkylamino mit jeweils 1 bis 6 Kohlenstoffatomen im Alkylteil; für Alkoxy mit 1 bis 6 Kohlenstoffatomen; für Alkenyloxy mit 3 bis 6 Kohlenstoffatomen; für Aralkoxy mit 6 bis 10 Kohlenstoffatomen im Arylteil und 1 bis 4 Kohlenstoffatomen im Alkylteil oder für gegebenenfalls durch Fluor, Chlor, Brom, Cyano, Nitro, Hydroxy, Mercapto, $C_1$—$C_4$-Alkoxy, $C_1$—$C_4$-Alkylthio, Halogen-$C_1$—$C_2$-alkoxy, Halogen-$C_1$—$C_2$-alkylthio, Amino, $C_1$—$C_4$-Alkylamino, Di-$C_1$—$C_4$-alkylamino, Hydroxycarbonyl, $C_1$—$C_4$-Alkoxycarbonyl, $C_1$—$C_4$-Alkylcarbonylamino, Morpholino oder $C_3$—$C_6$-Cycloalkyl substituiertes Alkyl mit 1 bis 20 Kohlenstoffatomen; für Alkenyl und Alkinyl mit jeweils 3 bis 6 Kohlenstoffatomen; für gegebenenfalls durch $C_1$—$C_2$-Alkyl, Fluor, Chlor, Brom oder Halogen-$C_1$—$C_2$-alkyl substituiertes Cycloalkyl mit 3 bis 8 Kohlenstoffatomen; für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_2$-alkyl, Halogen-$C_1$—$C_2$-alkoxy oder Halogen-$C_1$—$C_2$-alkylthio substituiertes Aralkyl mit 6 bis 10 Kohlenstoffatomen im Arylteil und 1 bis 4 Kohlenstoffatomen im Alkylteil sowie für gegebenenfalls durch Fluor, Chlor, Brom, Cyano, Nitro, $C_1$—$C_4$-Alkyl, $C_1$—$C_4$-Alkoxy, $C_1$—$C_4$-Alkylthio, Halogen-$C_1$—$C_4$-alkyl, Halogen-$C_1$—$C_4$-alkoxy, Halogen-$C_1$—$C_4$-alkylthio, Amino, $C_1$—$C_4$-Alkylamino oder Di-$C_1$—$C_4$-alkylamino substituiertes Furyl-$C_1$—$C_3$-alkyl, Thiophenyl-$C_1$—$C_3$-alkyl, Pyrazolyl-$C_1$—$C_3$-alkyl, Imidazolyl-$C_1$—$C_3$-alkyl, Pyrrolyl-$C_1$—$C_3$-alkyl, 1,2,4-Triazolyl-$C_1$—$C_3$-alkyl, 1,2,3-Triazolyl-$C_1$—$C_3$-alkyl, Pyridyl-$C_1$—$C_3$-alkyl, Pyrazinyl-$C_1$—$C_3$-alkyl, Pyrimidinyl-$C_1$—$C_3$-alkyl, Oxazolyl-$C_1$—$C_3$-alkyl, Isoxazolyl-$C_1$—$C_3$-alkyl, 1,2,4-Oxadiazolyl-$C_1$—$C_3$-alkyl, 1,3,4-Oxadiazolyl-$C_1$—$C_3$-alkyl, Thiazolyl-$C_1$—$C_3$-alkyl, Isothiazolyl-$C_1$—$C_3$-alkyl, 1,2,5-Thiadiazolyl-$C_1$—$C_3$-alkyl, 1,3,4-Thiadiazolyl-$C_1$—$C_3$-alkyl steht, dadurch gekennzeichnet, daß man Nitromethylen-Derivate der Formel (II)

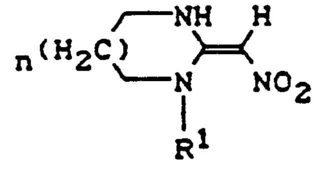

(II)

in welcher

$R^1$ und n die oben angegebenen Bedeutungen haben, mit Aminen der Formel (III)

$$R^2NH_2 \qquad (III)$$

in welcher

$R^2$ die oben angegebenen Bedeutungen hat,

in Gegenwart von mindestens der zweifachen molaren Menge Formaldehyd, gegebenenfalls in Gegenwart von sauren Katalysatoren und gegebenenfalls in Gegenwart von Verdünnungsmitteln umsetzt und gegebenenfalls an die erhaltenen Verbindungen physiologisch verträgliche Säuren addiert.

4. Schädlingsbekämpfungsmittel, gekennzeichnet durch einen Gehalt an indestens einem 1,2,3,6-Tetrahydro-5-nitropyrimidin-Derivat der Formel (I) gemäß Anspruch 1.

5. Insektizide und nematizide Mittel, gekennzeichnet durch einen Gehalt an mindestens einem 1,2,3,6-Tetrahydro-5-nitropyrimidin-Derivat der Formel (I) gemäß Anspruch 1.

6. Verfahren zur Bekämpfung von Insekten und/oder Nematoden, dadurch gekennzeichnet, daß man 1,2,3,6-Tetrahydro-5-nitropyrimidin-Derivat der Formel (I) gemäß Anspruch 1 auf Insekten und/oder Nematoden und/oder deren Lebensraum einwirken läßt.

7. Verwendung von 1,2,3,6-Tetrahydro-5-nitropyrimidin-Derivaten der Formel (I) gemäß Anspruch 1 zur Bekampfung von Insekten und/oder Nematoden.

8. Verfahren zur Herstellung von Schädlingsbekämpfungsmitteln, dadurch gekennzeichnet, daß man 1,2,3,6-Tetrahydro-5-nitropyrimidin-Derivat der Formel (I) gemäß Anspruch 1 mit Streckmitteln und/oder oberflächenaktiven Mitteln vermischt.

**Claims**

1. 1,2,3,6-Tetrahydro-5-nitro-pyrimidine derivatives of the formula (I)

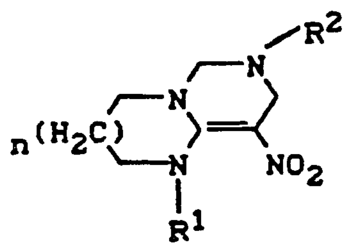

(I)

in which

n represents the numbers 0, 1 or 2,

$R^1$ represents aralkyl, having 6 to 10 carbon atoms in the aryl part and 1 to 4 carbon atoms in the alkyl part, which is optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_2$-alkyl, halogeno-$C_1$—$C_2$-alkoxy or halogeno-$C_1$—$C_2$-alkylthio, or represents furyl-$C_1$—$C_3$-alkyl, thiophenyl-$C_1$—$C_3$-alkyl, pyrazolyl-$C_1$—$C_3$-alkyl, imidazolyl-$C_1$—$C_3$-alkyl, pyrrolyl-$C_1$—$C_3$-alkyl, 1,2,4-triazolyl-$C_1$—$C_3$-alkyl, 1,2,3-triazolyl-$C_1$—$C_3$-alkyl, pyrimidinyl-$C_1$—$C_3$-alkyl, pyrazinyl-$C_1$—$C_3$-alkyl, pyridyl-$C_1$—$C_3$-alkyl, oxazolyl-$C_1$—$C_3$-alkyl, isoxazolyl-$C_1$—$C_3$-alkyl, 1,2,4-oxadiazolyl-$C_1$—$C_3$-alkyl, 1,3,4-oxadiazolyl-$C_1$—$C_3$-alkyl, thiazolyl-$C_1$—$C_3$-alkyl, isothiazolyl-$C_1$—$C_3$-alkyl, 1,2,5-thiadiazolyl-$C_1$—$C_3$-alkyl and 1,3,4-thiadiazolyl-$C_1$—$C_3$-alkyl which are optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_4$-alkyl, halogeno-$C_1$—$C_4$-alkoxy, halogeno-$C_1$—$C_4$-alkylthio, amino, $C_1$—$C_4$-alkylamino or di-$C_1$—$C_4$-alkylamino, and

$R^2$ represents dialkylamino having 1 to 6 carbon atoms in each alkyl part; alkoxy having 1 to 6 carbon atoms; alkenyloxy having 3 to 6 carbon atoms; aralkoxy having 6 to 10 carbon atoms in the aryl part and 1 to 4 carbon atoms in the alkyl part, or alkyl, having 1 to 20 carbon atoms, which is optionally substituted by fluorine, chlorine, bromine, cyano, nitro, hydroxyl, mercapto, $C_1$—$C_4$-alkoxy, $C_1$—$C_4$-alkylthio, halogeno-$C_1$—$C_2$-alkoxy, halogeno-$C_1$—$C_2$-alkylthio, amino, $C_1$—$C_4$-alkylamino, di-$C_1$—$C_4$-alkylamino, hydroxycarbonyl, $C_1$—$C_4$-alkoxycarbonyl, $C_1$—$C_4$-alkylcarbonylamino, morpholino or $C_3$—$C_6$-cycloalkyl; alkenyl and alkinyl each having 3 to 6 carbon atoms; cycloalkyl, having 3 to 8 carbon atoms, which is optionally substituted by $C_1$—$C_2$-alkyl, fluorine, chlorine, bromine or halogeno-$C_1$—$C_2$-alkyl; aralkyl, having 6 to 10 carbon atoms in the aryl part and 1 to 4 carbon atoms in the alkyl part, which is optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_2$-alkyl, halogeno-$C_1$—$C_2$-alkoxy or halogeno-$C_1$—$C_2$-alkylthio, and also represents furyl-$C_1$—$C_3$-alkyl, thiophenyl-$C_1$—$C_3$-alkyl, pyrazolyl-$C_1$—$C_3$-alkyl, imidazolyl-$C_1$—$C_3$-alkyl, pyrrolyl-$C_1$—$C_3$-alkyl, 1,2,4-triazolyl-$C_1$—$C_3$-alkyl, 1,2,3-triazolyl-$C_1$—$C_3$-alkyl, pyridyl-$C_1$—$C_3$-alkyl, pyrazinyl-$C_1$—$C_3$-alkyl, pyrimidinyl-$C_1$—$C_3$-alkyl, oxazolyl-$C_1$—$C_3$-alkyl, isoxazolyl-$C_1$—$C_3$-alkyl, 1,2,4-oxadiazolyl-$C_1$—$C_3$-alkyl, 1,3,4-oxadiazolyl-$C_1$—$C_3$-alkyl, thiazolyl-$C_1$—$C_3$-alkyl, isothiazolyl-$C_1$—$C_3$-alkyl, 1,2,5-thiadiazolyl-$C_1$—$C_3$-alkyl and 1,3,4-thiadiazolyl-$C_1$—$C_3$-alkyl which are optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_4$-alkyl, halogeno-$C_1$—$C_4$-alkoxy,

halogeno-$C_1$—$C_4$-alkylthio, amino, $C_1$—$C_4$-alkylamino or di-$C_1$—$C_4$-alkylamino, and the acid addition salts thereof.

2. 1,2,3,6-Tetrahydro-5-nitropyrimidine derivatives of the formula (I) according to Claim 1, in which

n represents the numbers 0 or 1,

$R^1$ represents benzyl and phenylethyl which are optionally substituted by fluorine, chlorine, bromine, cyano, nitro, methyl, ethyl, methoxy, ethoxy, methylthio, ethylthio, trifluoromethyl, trifluoromethoxy or trifluoromethylthio, and also represents thiophenylmethyl, pyrazolylmethyl, 1,2,4-triazolylmethyl, pyrazinylmethyl, pyrimidinylmethyl, pyridylmethyl, isoxazolylmethyl, oxazolylmethyl, thiazolylmethyl, 1,2,5-thiadiazolylmethyl or 1,3,4-thiadiazolylmethyl which are optionally substituted by fluorine, chlorine, bromine, cyano, nitro, methyl, ethyl, methoxy, methylthio, trifluoromethyl, trifluoromethoxy, trifluoromethylthio, dimethylamino or diethylamino, and

$R^2$ represents dialkylamino having 1 to 4 carbon atoms in each alkyl part; alkoxy having 1 to 4 carbon atoms; 1-prop-2-enyloxy; phenylmethoxy, phenylethoxy; alkyl, having 1 to 12 carbon atoms, which is optionally substituted by fluorine, chlorine, bromine, cyano, nitro, hydroxyl, mercapto, methoxy, ethoxy, methylthio, ethylthio, trifluoromethoxy, difluoromethoxy, trichloromethoxy, chlorodifluoromethoxy, trifluoromethylthio, difluoromethylthio, trichloromethylthio, chlorodifluoromethylthio, amino, methylamino, ethylamino, dimethylamino, di-ethylamino, hydroxycarbonyl, $C_1$—$C_2$-alkoxycarbonyl, $C_1$—$C_2$-alkylcarbonylamino or $C_3$—$C_6$-cycloalkyl; alkenyl and alkinyl each having 3 or 4 carbon atoms; cycloalkyl, having 3 to 6 carbon atoms, which is optionally substituted by fluorine, chlorine or trifluoromethyl; benzyl and phenethyl which are optionally substituted by fluorine, chlorine, bromine, cyano, nitro, methyl, ethyl, methoxy, ethoxy, methylthio, ethylthio, trifluoromethyl, trifluoromethoxy or trifluoromethylthio, and also represents furylmethyl, thiophenylmethyl, pyrrolylmethyl, pyrimidinylmethyl, thiazolylmethyl, pyrazolylmethyl, morpholinomethyl or morpholino-n-propyl which are optionally substituted by fluorine, chlorine, bromine, cyano, nitro, methyl, ethyl, methoxy, methylthio, trifluoromethyl, trifluoromethoxy, trifluoromethylthio, dimethylamino or diethylamino.

3. Process for the preparation of 1,2,3,6-tetrahydro-5-nitro-pyrimidine derivatives of the formula (I)

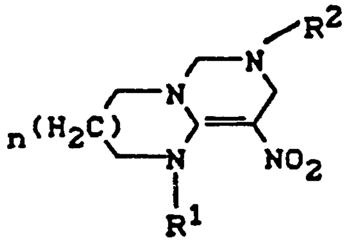

(I)

in which

n represents the numbers 0, 1 or 2,

$R^1$ represents aralkyl, having 6 to 10 carbon atoms in the aryl part and 1 to 4 carbon atoms in the alkyl part, which is optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_2$-alkyl, halogeno-$C_1$—$C_2$-alkoxy or halogeno-$C_1$—$C_2$-alkylthio, or represents furyl-$C_1$—$C_3$-alkyl, thiophenyl-$C_1$—$C_3$-alkyl, pyrazolyl-$C_1$—$C_3$-alkyl, imidazolyl-$C_1$—$C_3$-alkyl, pyrrolyl-$C_1$—$C_3$-alkyl, 1,2,4-triazolyl-$C_1$—$C_3$-alkyl, 1,2,3-triazolyl-$C_1$—$C_3$-alkyl, pyrimidinyl-$C_1$—$C_3$-alkyl, pyrazinyl-$C_1$—$C_3$-alkyl, pyridyl-$C_1$—$C_3$-alkyl, oxazolyl-$C_1$—$C_3$-alkyl, isoxazolyl-$C_1$—$C_3$-alkyl, 1,2,4-oxadiazolyl-$C_1$—$C_3$-alkyl, 1,3,4-oxadiazolyl-$C_1$—$C_3$-alkyl, thiazolyl-$C_1$—$C_3$-alkyl, isothiazolyl-$C_1$—$C_3$-alkyl, 1,2,5-thiadiazolyl-$C_1$—$C_3$-alkyl and 1,3,4-thiadiazolyl-$C_1$—$C_3$-alkyl which are optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_4$-alkyl, halogeno-$C_1$—$C_4$-alkoxy, halogeno-$C_1$—$C_4$-alkylthio, amino, $C_1$—$C_4$-alkylamino or di-$C_1$—$C_4$-alkylamino, and

$R^2$ represents dialkylamino having 1 to 6 carbon atoms in each alkyl part; alkoxy having 1 to 6 carbon atoms; alkenyloxy having 3 to 6 carbon atoms; aralkoxy having 6 to 10 carbon atoms in the aryl part and 1 to 4 carbon atoms in the alkyl part, or alkyl, having 1 to 20 carbon atoms, which is optionally substituted by fluorine, chlorine, bromine, cyano, nitro, hydroxyl, mercapto, $C_1$—$C_4$-alkoxy, $C_1$—$C_4$-alkylthio, halogeno-$C_1$—$C_4$-alkoxy, halogeno-$C_1$—$C_4$-alkylthio, amino, $C_1$—$C_4$-alkylamino, di-$C_1$—$C_4$-alkylamino, hydroxycarbonyl, $C_1$—$C_4$-alkoxycarbonyl, $C_1$—$C_4$-alkylcarbonylamino, morpholino or $C_3$—$C_6$-cycloalkyl; alkenyl and alkinyl each having 3 to 6 carbon atoms; cycloalkyl, having 3 to 8 carbon atoms, which is optionally substituted by $C_1$—$C_2$-alkyl, fluorine, chlorine, bromine or halogeno-$C_1$—$C_2$-alkyl; aralkyl, having 6 to 10 carbon atoms in the aryl part and 1 to 4 carbon atoms in the alkyl part, which is optionally substituted by fluorine, chlorine, bromine, cyano, nitro, $C_1$—$C_4$-alkyl, $C_1$—$C_4$-alkoxy, $C_1$—$C_4$-alkylthio, halogeno-$C_1$—$C_2$-alkyl, halogeno-$C_1$—$C_2$-alkoxy or halogeno-$C_1$—$C_2$-alkylthio, and also represents furyl-$C_1$—$C_3$-alkyl, thiophenyl-$C_1$—$C_3$-alkyl, pyrazolyl-$C_1$—$C_3$-alkyl, imidazolyl-$C_1$—$C_3$-alkyl, pyrrolyl-$C_1$—$C_3$-alkyl, 1,2,4-triazolyl-$C_1$—$C_3$-alkyl, 1,2,3-triazolyl-$C_1$—$C_3$-alkyl, pyridyl-$C_1$—$C_3$-alkyl, pyrazinyl-$C_1$—$C_3$-alkyl, pyrimidinyl-$C_1$—$C_3$-alkyl, oxazolyl-$C_1$—$C_3$-alkyl, isoxazolyl-$C_1$—$C_3$-alkyl, 1,2,4-oxadiazolyl-$C_1$—$C_3$-alkyl, 1,3,4-oxadiazolyl-$C_1$—$C_3$-alkyl, thiazolyl-$C_1$—$C_3$-alkyl, isothiazolyl-$C_1$—$C_3$-alkyl, 1,2,5-thiadiazolyl-$C_1$—$C_3$-alkyl and 1,3,4-thiadiazolyl-$C_1$—$C_3$-alkyl which are optionally substituted by fluorine, chlorine, bromine,

cyano, nitro, $C_1$—$C_4$-alkyl, $C_1$—$C_4$-alkoxy, $C_1$—$C_4$-alkylthio, halogeno-$C_1$—$C_4$-alkyl, halogeno-$C_1$—$C_4$-alkoxy, halogeno-$C_1$—$C_4$-alkylthio, amino, $C_1$—$C_4$-alkylamino or di-$C_1$—$C_4$-alkylamino characterized in that nitromethylene derivatives of the formula (II)

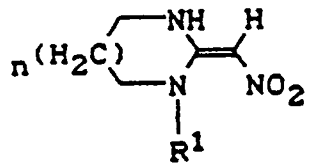
(II)

in which

$R^1$ and n have the abovementioned meanings, are reacted with amines of the formula (II)

$R^2NH_2$ (III)

in which

$R^2$ has the abovementioned meanings, in the presence of at least twice the molar amount of formaldehyde, if appropriate in the presence of acidic catalysts and if appropriate in the presence of diluents, and, if appropriate, physiologically acceptable acids are adducted to the compounds obtained.

4. Pesticides, characterized in that they contain at least one 1,2,3,6-tetrahydro-5-nitropyrimidine derivatives of the formula (I), according to Claim 1.

5. Insecticidal and nematicidal agents, characterized in that they contain at least one 1,2,3,6-tetrahydro-5-nitropyrimidine derivatives of the formula (I), according to Claim 1.

6. Process for combating insects and/or nematodes, characterized in that they contain at least one 1,2,3,6-tetrahydro-5-nitropyrimidine derivatives of the formula (I), according to Claim 1 are allowed to act on insects and/or nematodes and/or on their habitats.

7. Use of 1,2,3,6-tetrahydro-5-nitropyrimidine derivatives of the formula (I), according to Claim 1 for combating insects and/or nematodes.

8. Process for the preparation of pesticides, characterized in that 1,2,3,6-tetrahydro-5-nitropyrimidine derivatives of the formula (I), according to Claim 1 are mixed with extenders and/or surface-active agents.

**Revendications**

1. Dérivés de 1,2,3,6-tétrahydro-5-nitropyrimidine de formule (I)

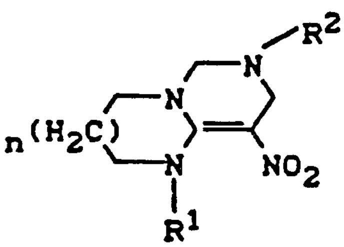
(I)

dans laquelle

n représente les nombres 0, 1 ou 2,

$R^1$ désigne un groupe aralkyle ayant 6 à 10 atomes de carbone dans la partie aryle et 1 à 4 atomes de carbone dans la partie alkyle, éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ ou $C_2$, halogénalkyle en $C_1$ ou $C_2$ halogénalkylthio en $C_1$ ou $C_2$ ainsi qu'un groupe furyl-(alkyle en $C_1$ ou $C_3$), thiophényle-(alkyle en $C_1$ à $C_3$), pyrazolyl-(alkyle en $C_1$ à $C_3$), imidazolyl-(alkyle en $C_1$ à $C_3$), pyrrolyl-(alkyle en $C_1$ à $C_3$), 1,2,4-triazolyl-(alkyle en $C_1$ à $C_3$), 1,2,3-triazolyl-(alkyle en $C_1$ à $C_3$), pyrimidinyl-(alkyle en $C_1$ à $C_3$), pyrazinyl-(alkyle en $C_1$ à $C_3$), pyridyl-(alkyle en $C_1$ à $C_3$), oxazolyl-(alkyle en $C_1$ à $C_3$), isoxazolyl-(alkyle en $C_1$ à $C_3$), 1,2,4-oxadiazolyl-(alkyle en $C_1$ à $C_3$), 1,3,4-oxadiazolyl-(alkyle en $C_1$ à $C_3$), thiazolyl-(alkyle en $C_1$ à $C_3$), isothiazolyl-(alkyle en $C_1$ à $C_3$), 1,2,5-thiadiazolyl-(alkyle en $C_1$ à $C_3$) et 1,3,4-thiadiazolyl-(alkyle en $C_1$ à $C_3$) éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkythio en $C_1$ à $C_4$, amino, alkylamino en $C_1$ à $C_4$ ou di-(alkyle en $C_1$ à $C_4$) amino et

$R^2$ est un groupe dialkylamino ayant 1 à 6 atomes de carbone dans chaque radical alkyle; un groupe alkoxy ayant 1 à 6 atomes de carbone; un groupe alcényloxy ayant 3 à 6 atomes de carbone; un groupe aralkoxy ayant 6 à 10 atomes de carbone dans la partie aryle et 1 à 4 atomes de carbone dans la partie alkyle ou un groupe alkyle de 1 à 20 atomes de carbone éventuellement substitué par du fluor, du chlore, du brome, un radical cyano, nitro, hydroxy, mercapto, alkoxy en $C_1$ à $C_4$, alkylthio en $C_1$ à $C_4$, halogénalkoxy en

$C_1$ ou $C_2$, halogénalkylthio en $C_1$ ou $C_2$, amino, alkylamino en $C_1$ à $C_4$, di-(alkyle en $C_1$ à $C_4$)amino, hydroxycarbonyle, (alkoxy en $C_1$ à $C_4$)carbonyle, (alkyle en $C_1$ à $C_4$)carbonylamino, morpholino ou cycloalkyle en $C_3$ à $C_6$; une groupe alcényle et un groupe alcynyle ayant chacun 3 à 6 atomes de carbone; un groupe cycloalkyle de 3 à 8 atomes de carbone eventuellement substitué par un radical alkyle en $C_1$ ou $C_2$, du fluor, du chlore, du brome ou un radical halogénalkyle en $C_1$ ou $C_2$; un groupe aralkyle ayant 6 à 10 atomes de carbone dans la partie aryle et 1 à 4 atomes de carbone dans la partie alkyle, éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ ou $C_2$, halogénalkoxy en $C_1$ ou $C_2$ ou halogénalkylthio en $C_1$ ou $C_2$ ainsi qu'un groupe furyl-(alkyle en $C_1$ à $C_3$), thiophényle-(alkyle en $C_1$ à $C_3$), pyrazolyl-(alkyle en $C_1$ à $C_3$), imidazolyl-(alkyle en $C_1$ à $C_3$), pyrrolyl-(alkyle en $C_1$ à $C_3$), 1,2,4-triazolyl-(alkyle en $C_1$ à $C_3$), 1,2,3-triazolyl-(alkyle en $C_1$ à $C_3$), pyridyl-(alkyle en $C_1$ à $C_3$), pyrazinyl-(alkyle en $C_1$ à $C_3$), pyrimidyl-(alkyle en $C_1$ à $C_3$), oxazolyl-(alkyle en $C_1$ à $C_3$), isoxazolyl-(alkyle en $C_1$ à $C_3$), 1,2,4-oxadiazolyl-(alkyle en $C_1$ à $C_3$), 1,3,4-oxadiazolyl-(alkyle en $C_1$ à $C_3$), thiazolyl-(alkyle en $C_1$ à $C_3$), isothiazolyl-(alkyle en $C_1$ à $C_3$), 1,2,5-thiadiazolyl-(alkyle en $C_1$ à $C_3$), 1,3,4-thiadiazolyl-(alkyle en $C_1$ à $C_3$) éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkythio en $C_1$ à $C_4$, amino, alkylamino en $C_1$ à $C_4$ ou di-(alkyle en $C_1$ à $C_4$) amino,

et leurs sels d'addition d'acides.

2. Dérivés de 1,2,3,6-térahydro-5-nitropyrimidine de formule (I) suivant la revendication 1, dans laquelle

n représente les nombres 0 ou 1,

$R^1$ représente les groupes benzyle et phényléthyle éventuellement substitués par du fluor, du chlore, du brome, un radical cyano, nitro, méthyle, éthyle, méthoxy, éthoxy, méthylthio, éthylthio, trifluorométhyle, trifluorométhoxy ou trifluorométhylthio, ainsi qu'un groupe thiophénylméthyle, pyrazolylméthyle, 1,2,4-triazolylméthyle, pyrazinylméthyle, pyrimidinylméthyle, pyridylméthyle, isoxazolylméthyle, oxazolylméthyle, thioazolylméthyle, 1,2,5-thiadiazolylméthyle ou 1,3,4-thiadiazolylméthyle éventuellement substitué par du fluor, du chlore, du brome, un radical cyano, nitro, méthyle, éthyle, méthoxy, méthylthio, trifluorométhyle, trifluorométhoxy, trifluorométhylthio, diméthylamino ou diéthylamino et

$R_2$ est un groupe dialkylamino ayant 1 à 4 atomes de carbone par radical alkyle; un groupe alkoxy ayant 1 à 4 atomes de carbone; un groupe 1-prop-2-ényloxy; phénylméthoxy, phényléthoxy; un groupe alkyle de 1 à 12 atomes de carbone éventuellement substitué par du fluor, du chlore, du brome, un radical cyano, nitro, hydroxy, mercapto, méthoxy, éthoxy, méthylthio, éthylthio, trifluorométhoxy, difluorométhoxy, trichlorométhoxy, chlorodifluorométhoxy, difluorométhoxy, trichlorométhoxy, chlorodifluorométhoxy, trifluorométhylthio, difluorométhylthio, trichlorométhylthio, chlorodifluorométhylthio, amino, méthylamino, éthylamino, diméthylamino, diéthylamino, hydroxycarbonyle, (alkoxy en $C_1$ ou $C_2$)-carbonyle, (alkyle en $C_1$ ou $C_2$)carbonylamino ou cycloalkyle en $C_3$ et $C_6$; un groupe alcényle et un groupe alcynyle ayant chacun 3 ou 4 atomes de carbone; un groupe cycloalkyle de 3 à 6 atomes de carbone éventuellement substitué par du fluor, du chlore ou un radical trifluorométhyle; un groupe benzyle et un groupe phényléthyle éventuellement substitués par du fluor, du chlore, du brome, un radical cyano, nitro, méthyle, éthyle, méthoxy, éthoxy, méthylthio, éthylthio, trifluorométhyle, trifluoromethoxy ou trifluorométhylthio, de même qu'un groupe furylméthyle, thiophénylméthyle, pyrrolylméthyle, pyrimidinylméthyle, thiazolylméthyle, pyrazolylméthyle, morpholinométhyle ou morpholino-n-propyle éventuellement substitué par du fluor, du chlore, du brome, un radical cyano, nitro, méthyle, éthyle, méthoxy, méthylthio, trifluorométhyle, trifluorométhoxy, trifluorométhylthio, diméthylamino ou diéthylamino.

3. Procédé de production de dérivés de 1,2,3,6-térahydro-5-nitro-pyrimidine de formule (I)

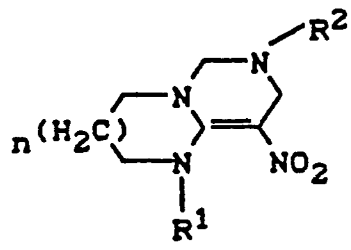 (I)

dans laquelle

n représente les nombres 0, 1 ou 2,

$R^1$ désigne un groupe aralkyle ayant 6 à 10 atomes de carbone dans la partie aryle et 1 à 4 atomes de carbone dans la partie alkyle, éventuellement substitué par du fluor, du chlore, du brome, un radical cyano, nitro, alkyle en $C_1$ à $C_4$, alkoxy en $C_1$ en $C_4$, alkylthio en $C_1$ à $C_4$, halogénalkyle en $C_1$ ou $C_2$, halogénalkyle en $C_1$ ou $C_2$, halogénalkoxy en $C_1$ ou $C_2$ ou halogénalkylthio en $C_1$ ou $C_2$ ainsi qu'un groupe furyl-(alkyle en $C_1$ ou $C_3$), thiophényl-(alkyle en $C_1$ à $C_3$), pyrazolyl-(alkyle en $C_1$ à $C_3$), imidazolyl-(alkyle en $C_1$ à $C_3$), pyrrolyl-

(alkyle en $C_1$ à $C_3$), 1,2,4-triazolyl-(alkyle en $C_1$ à $C_3$), 1,2,3-triazolyl-(alkyle en $C_1$ à $C_3$), pyrimidinyl-(alkyle en $C_1$ à $C_3$), pyrazinyl-(alkyle en $C_1$ à $C_3$), pyridyl-(alkyle en $C_1$ à $C_3$), oxazolyl-(alkyle en $C_1$ à $C_3$), isoxazolyl-(alkyle en $C_1$ à $C_3$), 1,2,4-oxadiazolyl-(alkyle en $C_1$ à $C_3$), 1,3,4-oxadiazolyl-(alkyle en $C_1$ à $C_3$), thiazolyl-(alkyle en $C_1$ à $C_3$), isothiazolyle-(alkyle en $C_1$ à $C_3$), 1,2,5-thiadiazolyl-(alkyle en $C_1$ à $C_3$) et 1,3,4-thiadiazolyl-(alkyle en $C_1$ à $C_3$) éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkythio en $C_1$ à $C_4$, amino, alkylamino en $C_1$ à $C_4$ ou di-(alkyle en $C_1$ à $C_4$)amino et

$R_2$ est un groupe dialkylamino ayant 1 à 6 atomes de carbone dans chaque radical alkyle; un groupe alkoxy ayant 1 à 6 atomes de carbone; un groupe alcényloxy ayant 3 à 6 atomes de carbone; un groupe alcényloxy ayant 6 à 10 carbon atoms de carbone dans la partie aryle et 1 à 4 atomes de carbone dans la partie alkyle ou un groupe alkyle de 1 à 20 atomes de carbone éventuellement substitué par du fluor, du chlore, du brome, un radical cyano, nitro, hydroxy, mercapto, alkoxy en $C_1$ à $C_4$, alkylthio en $C_1$ à $C_4$, halogénalkoxy en $C_1$ ou $C_2$, halogénalkylthio en $C_1$ ou $C_2$, amino, alkylamino en $C_1$ à $C_4$, di-(alkyle en $C_1$ à $C_4$)amino, hydroxycarbonyle, (alkoxy en $C_1$ à $C_4$)carbonyle, (alkyle en $C_1$ à $C_4$)carbonylamino, morpholino ou cycloalkyle en $C_3$ à $C_6$; une groupe alcényle et un groupe alcynyle ayant chacun 3 à 6 atomes de carbone; un groupe cycloalkyle de 3 à 8 atomes de carbone éventuellement substitué par un radical alkyle en $C_1$ ou $C_2$, du fluor, du chlore, du brome ou un radical halogénalkyle en $C_1$ ou $C_2$; un groupe aralkyle ayant 6 à 10 atomes de carbone dans la partie aryle et 1 à 4 atomes de carbone dans la partie alkyle, éventuellement substitué par du fluor, du chlore, du brome, un radical cyano, nitro, alkyle en $C_1$ à $C_4$, alkoxy en $C_1$ à $C_4$, alkylthio en $C_1$ à $C_4$, halogénalkyle en $C_1$ ou $C_2$, halogénalkoxy en $C_1$ ou $C_2$ ou halogénalkylthio en $C_1$ ou $C_2$ ainsi qu'un groupe furyl-(alkyle en $C_1$ à $C_3$), thiophényl-(alkyle en $C_1$ à $C_3$), pyrazolyl-(alkyle en $C_1$ à $C_3$), imidazolyl-(alkyle en $C_1$ à $C_3$), pyrrolyl-(alkyle en $C_1$ à $C_3$), 1,2,4-triazolyl-(alkyle en $C_1$ à $C_3$), 1,2,3-triazolyl-(alkyle en $C_1$ à $C_3$), pyridyl-(alkyle en $C_1$ à $C_3$), pyrazinyl-(alkyle en $C_1$ à $C_3$), pyrimidyl-(alkyle en $C_1$ à $C_3$), oxazolyl-(alkyle en $C_1$ à $C_3$), isoxazolyl-(alkyle en $C_1$ à $C_3$), 1,2,4-oxadiazolyl-(alkyle en $C_1$ à $C_3$), 1,3,4-oxadiazolyl-(alkyle en $C_1$ à $C_3$), thiazolyl-(alkyle en $C_1$ à $C_3$), isothiadiazolyl-(alkyle en $C_1$ à $C_3$), 1,2,5-thiadiazolyl-(alkyle en $C_1$ à $C_3$), 1,3,4-thiadiazolyl-(alkyle en $C_1$ à $C_3$) éventuellement substitué par du fluor, du chlore, un radical cyano, nitro, alkyle en $C_1$ à $C_4$, alkoxy en $C_1$ à $C_4$, alkylthio en $C_1$ à $C_4$, halogénalkyle en $C_1$ à $C_4$, halogénalkoxy en $C_1$ à $C_4$, halogénalkythio en $C_1$ à $C_4$, amino, alkylamino en $C_1$ à $C_4$ ou di-(alkyle en $C_1$ à $C_4$) amino,

caractérisé en ce qu'on fait réagir des dérivés nitrométhyléniques de formule (II)

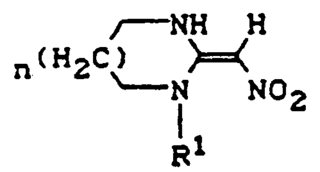

(II)

dans laquelle

$R_1$ et n ont les définitions indiquées ci-dessus, avec des amines de formule (III)

$$R^2NH_2 \qquad (III)$$

dans laquelle

$R^2$ a les définition indiquées ci-dessus, en présence d'au moins le double de la quantité molaire de formaldéhyde, le cas échéant en présence de catalysateurs acides et en la présence éventuellement de diluants et on additionne, le cas échéant sur les composés obtenus, des acides acceptables du point de vue physiologique.

4. Composition pesticide, caractérisée par une teneur en au moins un dérivé de 1,2,3,6-tétrahydro-5-nitropyrimidine de formule (I) suivant la revendication 1.

5. Compositions insecticides et nématicides, caractérisées par une teneur en au moins un dérivé de 1,2,3,6-tétrahydro-5-nitropyrimidine de formule (I) suivant la revendication 1.

6. Procédé pour combattre des insectes et/ou des nématodes, caractérisé en ce qu'un fait agir des dérivés de 1,2,3,6-tétrahydro-5-nitropyrimidine de formule (I) suivant la revendication 1 sur des insectes et/ou des nématodes et/ou sur leur habitat.

7. Utilisation de dérivés de 1,2,3,6-tétrahydro-5-nitropyrimidine de formule (I) suivant la revendication 1 pour combattre des insectes et/ou des nématodes.

8. Procédé de préparation de compositions pesticides, caractérisé en ce qu'on mélange des dérivés de 1,2,3,6-tétrahydro-5-nitropyrimidine de formule (I) suivant la revendication 1 avec des diluants et/ou des agents tensio-actifs.